# EUROPEAN PATENT APPLICATION

(11) **EP 3 932 946 A1**
(43) Date of publication of application: **05.01.2022**
(21) Application number: 20762642.5
(22) Date of filing: 25.02.2020
(51) Int. Cl.: C07K 16/22, A61P 35/00, G01N 33/574, A61K 39/00

(54) **ANTI-ANG2 ANTIBODY AND USE THEREOF**

(30) Priority: 25.02.2019 KR 20190021812
(71) Applicant: Pharmabcine Inc., Daejeon 34047 (KR)
(72) Inventor: NAM, Ju Ryoung, Cheongju-si Chungcheongbuk-do 28464 (KR); BYUN, Sang Soon, Daejeon 34907 (KR); KO, Jongil, Daejeon 34116 (KR); KIM, Do-yun, Daejeon 34046 (KR); LEE, Joo Hyoung, Seoul 06077 (KR); HA, Jung Min, Gunpo-si Gyeonggi-do 15823 (KR); PARK, Cheonho, Daejeon 34118 (KR); LEE, Eun-Ah, Daejeon 34088 (KR); LEE, Weon Sup, Daejeon 34120 (KR); YOO, Jin-San, Daejeon 34124 (KR)
(74) Representative: Mewburn Ellis LLP
(86) International application number: PCT/KR2020/002687
(87) International publication number: WO 2020/175886

(57) **Abstract**

The present disclosure relates to an antibody to inhibit function of Angiopoietin-2 (Ang-2) by binding specifically to Ang-2, and directed to an anti-Ang2 antibody, a nucleic acid encoding the same, a vector comprising the nucleic acid, a cell transformed with the vector, a method of preparing the same, an angiogenesis inhibitor comprising the same, a composition for treating a disease related with Angiopoietin-2 activation and/or overproduction, a composition for diagnosing a disease related with Angiopoietin-2 activation and/or overproduction, a composition for treating eye disease or a composition for preventing or treating a cancer, and a composition for combining an antibody binding to Ang2 with a drug other than the antibody binding to Ang2.

## Description

### [Technical Field]

The present disclosure relates to an antibody to inhibit function of Angiopoietin-2 (Ang-2) by binding specifically to Ang-2, and directed to an anti-Ang2 antibody, a nucleic acid encoding the same, a vector comprising the nucleic acid, a cell transformed with the vector, a method of preparing the same, an angiogenesis inhibitor comprising the same, a composition for treating a disease related with Angiopoietin-2 activation and/or overproduction, a composition for diagnosing a disease related with Angiopoietin-2 activation and/or overproduction, a composition for treating eye disease or a composition for preventing or treating a cancer, and a composition for combining an antibody binding to Ang2 with a drug other than the antibody binding to Ang2.

### [Background Art]

Angiogenesis refers to a mechanism involving the growth of new blood vessels from pre-existing vessels, and is known to play a vital role in the formation of organs, normal biological growth, wound healing, and the like. It is also known to play an important role in tumor growth and metastasis, and abnormal angiogenesis is known to play a critical role in diseases such as tumor growth and metastasis, age-related macular degeneration, diabetic retinopathy, psoriasis, rheumatoid arthritis, and chronic inflammation.

Thus, factors involved in angiogenesis have become an important target for the development of new therapeutic agents for diseases such as cancer, and as the number of diabetes patients increases rapidly due to aging and westernized eating habits, the number of patients with neovascular eye diseases is rapidly increasing. Examples of major eye diseases include age-related macular degeneration (AMD), diabetic retinopathy (DR), and diabetic macular edema (DME). In particular, macular degeneration and diabetic retinopathy are the leading causes of blindness worldwide.

There are many factors related to the progression of age-related macular degeneration, but it is known to be associated with oxidative stress, inflammatory response, and angiogenesis. However, vascular endothelial growth factor (VEGF) is widely considered to be the predominant factor. Attempts have been made to develop, as a therapeutic agent, a VEGF inhibitor using a monoclonal antibody, an antibody fragment, or a fusion protein, and aflibercept and ranibizumab are used as representative drugs. The mechanism of action of these drugs is known to induce angiogenesis inhibition by inhibition of VEGF signaling. However, it is known that 10-15% of patients to whom these drugs are administered do not respond to existing treatments. This is because it is known that existing anti-VEGF treatment suppresses only pathological angiogenesis, whereas other pathways of angiogenic factors influence disease progression. Angiopoietin 2 (ANG2) is known as a cytokine that binds to the Tie2 receptor present in endothelial cells of the vascular wall and promotes angiogenesis. Through previous animal experiments and clinical trials, it is known that, by suppressing the inhibition of ANG2 signaling, the formation of blood vessels in tumors is inhibited, and thus an anti-cancer effect is exhibited. Moreover, ANG expression is known to be high in the waterproof fluid of the eyeballs of patients with age-related macular degeneration. Thus, it is expected that the development of an anti-ANG2 therapeutic agent along with an anti-VEGF therapeutic agent and combined therapy will be helpful in the treatment of macular degeneration. Accordingly, the applicant of the present disclosure focused on Ang-2 to develop therapeutic agents for age-related macular degeneration and diabetic retinopathy.

Angiopoietin-2 (Ang2) is an antagonistic ligand of the receptor Tie2, present in vascular endothelial cells, and inhibits signal transduction by Tie2 by competing with angiopoietin-1 (Ang1), which is a Tie2 agonist, for Tie2 binding, and Ang1, which is a ligand for activating the Tie2 receptor, acts as a key regulator for maintaining the stabilization of blood vessels by maintaining the barrier function of vascular endothelial cells. In the state of overexpression or inflammation of VEGF, vascular endothelial cells are activated, and vascular permeability increases.

In this regard, Ang1 promotes junctional integrity of vascular endothelial cells, thereby inducing the stabilization of vascular endothelial cells and reducing vascular permeability, whereas increased Ang2 in activated vascular endothelial cells binds to Tie-2, thereby being involved in the migration of vascular endothelial cells and tip formation. Consequently, the formation of new blood vessels is promoted.

In the case of diabetic retinopathy, it has been found that PDGF signaling is essential for the formation and maturation of the blood-retinal barrier by regulating vascular peripheral cells, and it has been proven that the loss of vascular peripheral cells in adult retinal vessels increases the response of vascular endothelial cells to VEGF-A, thereby activating the FOXO1-Ang2 loop, resulting in exacerbated diabetic retinopathy. In other words, it is determined that inducing Ang2 blocking and Tie2 activation will make it possible to develop new treatments for diabetic retinopathy.

In addition, Ang-2 also contributes to the formation of new blood vessels in cancer tissue. In order to form new blood vessels in cancer tissue, a cooption occurs in which cancer cells select existing blood vessels. Thereafter, vascular degeneration occurs, which destroys the function of existing blood vessels by the Ang-2 pathway. Due to the degeneration of existing blood vessels, the environment in cancer tissue becomes a hypoxic environment, providing conditions for the formation of new blood vessels. Under the above conditions, overexpression of vascular endothelial cell growth factor (VEGF) is induced, and as mentioned above, angiogenesis is induced. For this reason, Ang-2 has been a major target for the development of anticancer drugs through angiogenesis inhibitors.

Under the technical background mentioned above, the inventors of the present application have endeavored to develop anti-Ang2 antibodies. As a result, the inventors developed an anti-Ang2 antibody that exhibits desired ability to bind to Ang2, and confirmed that such an anti-Ang2 antibody can serve as a targeted immune anti-cancer agent or a therapeutic agent for ophthalmic diseases, and thus completed the present disclosure.

### [Disclosure]

### [Technical Problem]

Therefore, the present disclosure has been made in view of the above problems, and it is an object of the present disclosure to provide a novel antibody against Ang2 or an antigen-binding fragment thereof.

It is another object of the present disclosure to provide a nucleic acid encoding the antibody or the antigen-binding fragment thereof.

It is a further object of the present disclosure to provide a vector including the nucleic acid, a cell transformed with the vector, and a method of constructing the same.

It is a further object of the present disclosure to provide an angiogenesis inhibitor including the antibody or the antigen-binding fragment thereof, and a composition for the treatment of diseases related to angiopoietin-2 activation and/or overproduction.

It is a further object of the present disclosure to provide an angiogenesis inhibitor including the antibody or the antigen-binding fragment thereof, and a composition for diagnosing diseases related to angiopoietin-2 activation and/or overproduction.

It is a further object of the present disclosure to provide a composition for the prevention or treatment of eye diseases including the antibody or the antigen-binding fragment thereof.

It is a further object of the present disclosure to provide a composition for preventing or treating tumors or cancer including the antibody or the antigen-binding fragment thereof.

It is a further object of the present disclosure to provide a composition for co-administration with an anti-Ang2 antibody, including the antibody or the antigen-binding fragment thereof.

### [Technical Solution]

In accordance with an aspect of the present disclosure, the above and other objects can be accomplished by the provision of an antibody binding to angiopoietin-2 (Ang2) or an antigen-binding fragment thereof, including:
a heavy chain variable region including a heavy chain CDR1 selected from the group consisting of SEQ ID NOS: 1, 7, 13, 19, and 25, a heavy chain CDR2 selected from the group consisting of SEQ ID NOS: 2, 8, 14, 20, and 26, and a heavy chain CDR3 selected from the group consisting of SEQ ID NOS: 3, 9, 15, 21, 27, 51, 52, and 53; and
a light chain variable region including a light chain CDR1 selected from the group consisting of SEQ ID NOS: 4, 10, 16, 22, and 28, a light chain CDR2 selected from the group consisting of SEQ ID NOS: 5, 11, 17, 23, and 29, and a light chain CDR3 selected from the group consisting of SEQ ID NOS: 6, 12, 18, 24, and 30.

In accordance with another aspect of the present disclosure, there is provided a nucleic acid encoding the antibody or the antigen-binding fragment thereof.

In accordance with a further aspect of the present disclosure, there is provided a vector including the nucleic acid.

In accordance with a further aspect of the present disclosure, there is provided a cell transformed with the vector.

In accordance with a further aspect of the present disclosure, there is provided a method of producing the antibody or the antigen-binding fragment thereof, including the following processes: (a) culturing the cells; and (b) recovering the antibody or the antigen-binding fragment thereof from the cultured cells.

The present disclosure also provides an angiogenesis inhibitor including the antibody or the antigen-binding fragment thereof and a composition for the treatment of diseases related to angiopoietin-2 activation and/or overproduction. The present disclosure also provides an angiogenesis inhibitor including the antibody or the antigen-binding fragment thereof and a composition for the diagnosis of diseases related to angiopoietin-2 activation and/or overproduction. The present disclosure also provides a composition for the prevention or treatment of tumors or cancer including the antibody or the antigen-binding fragment thereof. The present disclosure also provides a composition for co-administration with an anti-Ang2 antibody including the antibody or the antigen-binding fragment thereof.

### [Description of Drawings]

The above and other objects, features and other advantages of the present disclosure will be more clearly understood from the following detailed description taken in conjunction with the accompanying drawings, in which:
FIG. 1 illustrates results confirming that a selected monoclonal scFv phage had the ability to inhibit Ang2/Tie2 binding;
FIG. 2 illustrates SDS-PAGE results under reducing and non-reducing conditions for products obtained after temporary expression and purification of selected anti-Ang2 antibodies;
FIG. 3 illustrates ELISA results obtained by evaluating the binding of temporarily expressed and purified anti-Ang2 antibodies to human and mouse Ang2 and Ang1;
FIG. 4 illustrates results showing the abilities of selected anti-Ang2 antibodies to neutralize human and mouse Ang2/Tie2 binding;
FIG. 5 illustrates results showing the abilities of selected anti-Ang2 antibodies to neutralize Ang2/integrin binding;
FIG. 6 illustrates results showing that selected anti-Ang2 antibodies can inhibit Ang2/Tie2 signaling;
FIG. 7 illustrates results confirming the purity of a selected anti-Ang2 scFv antibody expressed in E. *coli* according to a purification process;
FIG. 8 illustrates ELISA results obtained by evaluating binding of an anti-Ang2 scFv antibody expressed in *E. coli* to a human Ang2 protein;
FIGS. 9 to 11 illustrate the results of confirming the *in-vivo* efficacy of a selected anti-Ang2 ScFv antibody in a CNV mouse model; and
FIG. 12 illustrates results showing the antitumor effect of an anti-Ang2 antibody using a human-derived triple-negative breast cancer model.

### [Detailed Description and Exemplary Embodiments]

Reference will now be made in detail to the preferred embodiments of the present disclosure, examples of which are illustrated in the accompanying drawings. Wherever possible, the same reference numbers will be used throughout the drawings to refer to the same or like parts.

Unless defined otherwise, all technical and scientific terms used herein have the same meanings as commonly understood by one of ordinary skill in the art to which the present disclosure pertains. In general, the nomenclature used herein and experimental methods described below are well known and commonly used in the art.

An embodiment of the present disclosure relates to an antibody binding to angiopoietin-2 (Ang2) or an antigen-binding fragment thereof, including:
a heavy chain variable region including a heavy chain CDR1 selected from the group consisting of SEQ ID NOS: 1, 7, 13, 19, and 25, a heavy chain CDR2 selected from the group consisting of SEQ ID NOS: 2, 8, 14, 20, and 26, and a heavy chain CDR3 selected from the group consisting of SEQ ID NOS: 3, 9, 15, 21, 27, 51, 52, and 53; and
a light chain variable region including a light chain CDR1 selected from the group consisting of SEQ ID NOS: 4, 10, 16, 22, and 28, a light chain CDR2 selected from the group consisting of SEQ ID NOS: 5, 11, 17, 23, and 29, and a light chain CDR3 selected from the group consisting of SEQ ID NOS: 6, 12, 18, 24, and 30.

As used herein, the term "antibody" refers to an anti-Ang2 antibody that specifically binds to Ang2. Not only a complete antibody form that specifically binds to Ang2 but also an antigen-binding fragment of the antibody molecule fall within the scope of the present disclosure.

A complete antibody is a structure having two full-length light chains and two full-length heavy chains, wherein each light chain is linked to a heavy chain by disulfide bonds. The heavy chain constant region is of a gamma (γ), mu (µ), alpha (α), delta (δ), or epsilon (ε) type, which can be further categorized as gamma 1 (γ1), gamma 2 (γ2), gamma 3 (γ3), gamma 4 (γ4), alpha 1 (α1), or alpha 2 (α2). The light chain constant region is of either a kappa (κ) or lambda (λ) type.

An antigen-binding fragment of an antibody or an antibody fragment means a fragment having an antigen-binding function, and includes Fab, Fab', F(ab')2, and Fv. Among antibody fragments, Fab is a structure having variable regions of a light chain and a heavy chain, constant regions of the light chain, and a first constant region CH1 of the heavy chain, and has one antigen-binding site. Fab' is different from Fab in that Fab' has a hinge region including at least one cysteine residue at the C-terminus of a heavy chain CH1 domain. The F(ab')2 fragment is produced, whereby cysteine residues of Fab' are joined by a disulfide bond at the hinge region. Fv is the minimal antibody fragment having only heavy chain variable regions and light chain variable regions. Two-chain Fv may have a structure in which heavy chain variable regions are linked to light chain variable regions by a noncovalent bond, and single-chain Fv (scFv) generally has a dimer structure, as in the two-chain Fv, in which heavy chain variable regions are covalently bound to light chain variable regions via a peptide linker or heavy and light chain variable regions are directly linked to each other at the C-terminus thereof. The antigen-binding fragment may be obtained using protease (e.g., a whole antibody is restriction-digested with papain to obtain Fab, and is digested with pepsin to obtain F(ab')₂ fragments), and may also be prepared by a genetic recombination technique.

In one embodiment, the antibody according to the present disclosure is in the form of Fv (e.g., scFv) or in a complete antibody form. In addition, the heavy chain constant region can be any isotype selected from gamma (γ), mu (µ), alpha (α), delta (δ), and epsilon (ε). For example, the constant region is gamma 1 (IgG1), gamma 3 (IgG3), or gamma 4 (IgG4). The light chain constant region may be of a kappa or lambda type.

The term "heavy chain" used herein includes full-length heavy chains including a variable region VH including amino acid sequences having variable region sequences to allow antigens to have specificity and three constant domains CH1, CH2, and CH3, and fragments thereof. The term "light chain" used herein includes full-length light chains including a variable region VL including amino acid sequences having variable region sequences to allow antigens to have specificity and a constant region CL, and fragments thereof.

The antibody of the present disclosure may be a monoclonal antibody, a bispecific antibody, a human antibody, a humanized antibody, a chimeric antibody, a single-chain Fvs (scFV) fragment, a single-chain antibody, a Fab fragment, a F(ab') fragment, a disulfide-bond Fvs (sdFV) fragment, an anti-idiotype (anti-Id) antibody, and epitope-binding fragments of these antibodies, but the present disclosure is not limited thereto.

The monoclonal antibody refers to an antibody obtained from a substantially homogeneous antibody population, i.e., identical antibodies except for possible naturally occurring mutations where individual antibodies in the population may be present in trace amounts. Monoclonal antibodies are highly specific to a single antigenic site. Unlike conventional (polyclonal) antibody preparations including different antibodies for different determinants (epitopes), each monoclonal antibody is induced against a single epitope on the antigen.

"Epitope" refers a protein determinant to which an antibody can specifically bind. Epitopes usually consist of a group of chemically active surface molecules, for example, amino acid or sugar side chains, and generally have specific three-dimensional structural characteristics as well as specific charge properties. Conformational epitopes and non-conformational epitopes are distinguished in that binding to the former is lost in the presence of a denaturing solvent, but not to the latter.

The non-human (e.g., murine) antibody in "humanized" form is a chimeric antibody containing a minimal sequence derived from non-human immunoglobulin. In most cases, a humanized antibody is a non-human species (donor antibody) that retains the desired specificity, affinity, and ability for residues from the hypervariable region of a recipient, for example, a human immunoglobulin (receptor antibody) replaced with a residue from a hypervariable region of a mouse, rat, rabbit, or non-human.

The "human antibody" is a molecule derived from human immunoglobulin, and means that all amino acid sequences constituting an antibody, including complementarity-determining regions and framework regions, consist of human immunoglobulin.

The human antibody includes not only "chimeric" antibodies, in which part of the heavy chain and/or light chain has a sequence identical or homologous to the corresponding sequence of an antibody derived from a specific species or belonging to a specific antibody class or subclass, while the remaining chain(s) is/are identical or homologous to an antibody derived from another species or an antibody belonging to another antibody class or subclass, but also fragments thereof that exhibit desired biological activities.

"Antibody variable domain" as used herein refers to light and heavy chain portions of an antibody molecule including amino acid sequences of complementarity-determining regions (CDRs; CDR1, CDR2, and CDR3) and framework regions (FR). VH refers to the variable domain of a heavy chain. VL refers to the variable domain of a light chain.

"Complementarity-determining regions" (CDR; CDR1, CDR2, and CDR3) refers to amino acid residues of an antibody variable domain, which are necessary for antigen binding. Each variable domain typically has three CDR regions identified as CDR1, CDR2, and CDR3. The present disclosure includes a heavy chain variable region including heavy chain CDR3 of SEQ ID NO: 1 and a light chain variable region including light chain CDR3 of SEQ ID NO: 2.

In the present disclosure, the antibody binding to Ang2 or the antigen-binding fragment thereof may include:
a heavy chain variable region including a heavy chain CDR1 of SEQ ID NO: 1, a heavy chain CDR2 of SEQ ID NO: 2, and a heavy chain CDR3 of SEQ ID NO: 3, and a light chain variable region including a light chain CDR1 of SEQ ID NO: 4, a light chain CDR2 of SEQ ID NO: 5, and a light chain CDR3 of SEQ ID NO: 6;
a heavy chain variable region including a heavy chain CDR1 of SEQ ID NO: 7, a heavy chain CDR2 of SEQ ID NO: 8, and a heavy chain CDR3 of SEQ ID NO: 9, and a light chain variable region including a light chain CDR1 of SEQ ID NO: 10, a light chain CDR2 of SEQ ID NO: 11, and a light chain CDR3 of SEQ ID NO: 12;
a heavy chain variable region including a heavy chain CDR1 of SEQ ID NO: 13, a heavy chain CDR2 of SEQ ID NO: 14, and a heavy chain CDR3 of SEQ ID NO: 15, and a light chain variable region including a light chain CDR1 of SEQ ID NO: 16, a light chain CDR2 of SEQ ID NO: 17, and a light chain CDR3 of SEQ ID NO: 18;
a heavy chain variable region including a heavy chain CDR1 of SEQ ID NO: 19, a heavy chain CDR2 of SEQ ID NO: 20, and a heavy chain CDR3 of SEQ ID NO: 21, and a light chain variable region including a light chain CDR1 of SEQ ID NO: 22, a light chain CDR2 of SEQ ID NO: 23, and a light chain CDR3 of SEQ ID NO: 24;
a heavy chain variable region including a heavy chain CDR1 of SEQ ID NO: 25, a heavy chain CDR2 of SEQ ID NO: 26, and a heavy chain CDR3 of SEQ ID NO: 27, and a light chain variable region including a light chain CDR1 of SEQ ID NO: 28, a light chain CDR2 of SEQ ID NO: 29, and a light chain CDR3 of SEQ ID NO: 30;
a heavy chain variable region including a heavy chain CDR1 of SEQ ID NO: 13, a heavy chain CDR2 of SEQ ID NO: 14, and a heavy chain CDR3 of SEQ ID NO: 51, and a light chain variable region including a light chain CDR1 of SEQ ID NO: 16, a light chain CDR2 of SEQ ID NO: 17, and a light chain CDR3 of SEQ ID NO: 18;
a heavy chain variable region including a heavy chain CDR1 of SEQ ID NO: 13, a heavy chain CDR2 of SEQ ID NO: 14, and a heavy chain CDR3 of SEQ ID NO: 52, and a light chain variable region including a light chain CDR1 of SEQ ID NO: 16, a light chain CDR2 of SEQ ID NO: 17, and a light chain CDR3 of SEQ ID NO: 18; or
a heavy chain variable region including a heavy chain CDR1 of SEQ ID NO: 13, a heavy chain CDR2 of SEQ ID NO: 14, and a heavy chain CDR3 of SEQ ID NO: 53, and a light chain variable region including a light chain CDR1 of SEQ ID NO: 16, a light chain CDR2 of SEQ ID NO: 17, and a light chain CDR3 of SEQ ID NO: 18.

"Framework region (FR)" is a variable domain residue other than CDR residues. Each variable domain typically has four FRs, identified as FR1, FR2, FR3, and FR4.

An "Fv" fragment is an antibody fragment that contains a complete antibody recognition and binding site. This region consists of a dimer of one heavy chain variable domain and one light chain variable domain via tight covalent binding, e.g., scFv.

An "Fab" fragment contains the variable and constant domains of a light chain and the variable and first constant domains (CH1) of a heavy chain. F(ab')2 antibody fragments generally include a pair of Fab fragments that are covalently linked near the carboxy terminus thereof by hinge cysteines therebetween.

"Single-chain Fv" or "scFv" antibody fragments include the VH and VL domains of an antibody, which are present in a single polypeptide chain. An Fv polypeptide may further include a polypeptide linker between the VH domain and the VL domain that allows scFv to form a desired structure for antigen binding.

Anti-Ang2 antibodies may include a single chain or a double chain. Functionally, the binding affinity of anti-Ang2 antibodies is in the range of 10⁻⁵ M to 10⁻¹² M. For example, the binding affinity of anti-Ang2 antibodies ranges from 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10-⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M.

The antibody binding to Ang2 or the antigen-binding fragment thereof may include a heavy chain variable region selected from the group consisting of SEQ ID NOS: 32, 36, 40, 44, 48, 55, 57, 59, and 61. In addition, the antibody binding to Ang2 or the antigen-binding fragment thereof may include a light chain variable region selected from the group consisting of SEQ ID NOS: 34, 38, 42, 46, and 50.

In particular, in order to develop productive and highly concentrated formulations of anti-Ang2 antibodies, a mutation was induced in the framework portion of a heavy chain variable region for the purpose of enhancing productivity and solubility. A mutant was produced by converting valine, which is 12nd amino acid of a heavy chain variable region, into serine. Accordingly, an antibody including the heavy chain variable region of SEQ ID NO: 61 was used in an experiment. As a result, it was confirmed that enhanced productivity and solubility were exhibited.

In a specific embodiment according to the present disclosure, the antibody binding to Ang2 or the antigen-binding fragment thereof may include:
a heavy chain variable region of SEQ ID NO: 32 and a light chain variable region of SEQ ID NO: 34;
a heavy chain variable region of SEQ ID NO: 36 and a light chain variable region of SEQ ID NO: 38;
a heavy chain variable region of SEQ ID NO: 40 and a light chain variable region of SEQ ID NO: 42;
a heavy chain variable region of SEQ ID NO: 44 and a light chain variable region of SEQ ID NO: 46;
a heavy chain variable region of SEQ ID NO: 48 and a light chain variable region of SEQ ID NO: 50;
a heavy chain variable region of SEQ ID NO: 55 and a light chain variable region of SEQ ID NO: 42;
a heavy chain variable region of SEQ ID NO: 57 and a light chain variable region of SEQ ID NO: 42;
a heavy chain variable region of SEQ ID NO: 59 and a light chain variable region of SEQ ID NO: 42; or
a heavy chain variable region of SEQ ID NO: 61 and a light chain variable region of SEQ ID NO: 42.

"Phage display" is a technique for displaying a variant polypeptide as a fusion protein with at least a portion of an envelope protein on the surface of a phage, e.g., fibrous phage particles. The usefulness of phage display lies in the fact that it can target a large library of randomized protein variants and quickly and efficiently classify sequences that bind to a target antigen with high affinity. Displaying peptide and protein libraries using phages has been used to screen millions of polypeptides to identify polypeptides with specific binding properties.

Phage display technology has provided a powerful tool for generating and screening new proteins that bind to specific ligands (e.g., antigens). Using phage display technology, a large library of protein variants can be generated, and sequences that bind with high affinity to a target antigen can be quickly sorted. A nucleic acid encoding a variant polypeptide is fused with a viral envelope protein, such as a nucleic acid sequence encoding a gene III protein or gene VIII protein. A monovalent phage display system has been developed, in which a nucleic acid sequence encoding a protein or polypeptide is fused with a nucleic acid sequence encoding a portion of the gene III protein. In the monovalent phage display system, gene fusion is expressed at low levels, and the wild type gene III protein is also expressed, thus maintaining particle infectivity.

Demonstrating the expression of peptides on the surface of a fibrous phage and the expression of functional antibody fragments in the periplasm of *E. coli* is important in developing antibody phage display libraries. Libraries of antibodies or antigen-binding polypeptides have been prepared in a number of ways, for example by altering a single gene by inserting a random DNA sequence or cloning a related gene family. The library can be screened for expression of antibodies or antigen-binding proteins having desired characteristics.

Phage display technology has several advantages over conventional hybridoma and recombinant methods for producing antibodies with desired characteristics. This technique makes it possible to generate large antibody libraries with various sequences in a short time without using animals. The production of hybridomas or humanized antibodies may require several months. In addition, since no immunity is required, the phage antibody library can generate antibodies against antigens that are toxic or have low antigenicity. Phage antibody libraries can also be used to generate and identify new therapeutic antibodies.

Techniques may be used to generate human antibodies from immunized or non-immunized humans, germline sequences, or unsensitized B cell Ig repertories using phage display libraries. Various lymphoid tissues can be used to prepare unsensitized or non-immune antigen-binding libraries.

Techniques for identifying and isolating high-affinity antibodies from phage display libraries are important for the isolation of new therapeutic antibodies. Separation of the high-affinity antibody from the library can depend on the size of the library, the production efficiency among bacterial cells, and the diversity of the library. The size of a library is reduced by inadequate folding of an antibody or antigen-binding protein and inefficient production due to the presence of stop codons. Expression in bacterial cells can be suppressed if an antibody or antigen-binding domain is not properly folded. Expression can be improved by alternatively mutating the surface of a variable/constant interface or selected CDR residues. The sequence of the framework region is one element for providing proper folding when generating antibody phage libraries in bacterial cells.

It is important to generate various libraries of antibodies or antigen-binding proteins in high-affinity antibody isolation. CDR3 regions have often been found to participate in antigen binding. CDR3 regions on the heavy chain vary considerably in size, sequence, and structural conformation, and thus can be used to prepare various libraries.

In addition, diversity can be generated by randomizing the CDR regions of variable heavy and light chains using all 20 amino acids at each position. Using all 20 amino acids can result in a variant antibody sequence with great diversity and an increased chance of identifying new antibodies.

The antibody or antibody fragment of the present disclosure may include the sequence of the anti-Ang2 antibody of the present disclosure described herein, as well as biological equivalents thereto, within the range capable of specifically recognizing Ang2. For example, additional changes can be made to the amino acid sequence of the antibody to further improve the binding affinity and/or other biological properties of the antibody. Such modifications include, for example, deletion, insertion, and/or substitution of amino acid sequence residues of the antibody. These amino acid variations are made based on the relative similarity of amino acid side-chain substituents, for example, hydrophobicity, hydrophilicity, charge, size, and the like. Analysis of the size, shape, and type of amino acid side-chain substituents shows that arginine, lysine, and histidine are all positively charged residues, alanine, glycine, and serine have similar sizes, and phenylalanine, tryptophan, and tyrosine have similar shapes. Therefore, based on these considerations, arginine, lysine, and histidine; alanine, glycine, and serine; and phenylalanine, tryptophan, and tyrosine may be considered as biologically functional equivalents.

In view of the above-described variations with biologically equivalent activity, the antibody of the present disclosure or a nucleic acid molecule encoding the same is interpreted to also include a sequence showing substantial identity with the sequence set forth in sequence ID numbers. The substantial identity means a sequence with at least 90% homology, and most preferably at least 95% homology, at least 96% homology, at least 97% homology, at least 98% homology, or at least 99% homology, when the above-described sequence of the present disclosure is aligned to correspond to another arbitrary sequence as closely as possible, and the aligned sequence is analyzed using a commonly used algorithm. Alignment methods for sequence comparison are known in the art. NCBI Basic Local Alignment Search Tool (BLAST) can be accessed from NCBI or the like, and can be used in conjunction with sequence analysis programs such as blastp, blasm, blastx, tblastn, and tblastx on the Internet. BLAST can be accessed at www.ncbi.nlm.nih.gov/BLAST/. How to compare sequence homology using this program can be confirmed at www.ncbi.nlm.nih.gov/BLAST/blast_help.html.

Based on this, the antibody or antigen-binding fragment thereof of the present disclosure may have homology of 90%, 91%, 92%, 93%, 94%, 95%, 96%, 97%, 98%, 99% or more compared to the sequence specified in the specification. This homology may be determined by sequence comparison and/or alignment by methods known in the art. For example, sequence comparison algorithms (i.e., BLAST or BLAST 2.0), manual alignment, or visual inspection may be used to determine the sequence homology of nucleic acids or proteins of the invention in the form of a percentage.

Another embodiment of the present disclosure relates to a nucleic acid encoding the antibody or the antigen-binding fragment thereof.

An antibody or antigen-binding fragment thereof may be recombinantly produced by isolating the nucleic acid encoding the antibody or antigen-binding fragment thereof of the present disclosure. The nucleic acid is isolated and inserted into a replicable vector for further cloning (amplification of DNA) or further expression. Based on this, another embodiment of the present disclosure relates to a vector including the nucleic acid.

"Nucleic acid" has a meaning comprehensively including DNA (gDNA and cDNA) and RNA molecules, and a nucleotide, which is a basic structural unit in nucleic acids, includes not only natural nucleotides, but also sugar or analogues with modified base sites. The sequence of a nucleic acid encoding heavy chain and light chain variable regions of the present disclosure may be modified. Such modifications include addition, deletion, or non-conservative or conservative substitutions of nucleotides.

In a specific embodiment according to the present disclosure, the nucleic acid may include a nucleic acid encoding a heavy chain variable region selected from the group consisting of SEQ ID NOS: 31, 35, 39, 43, 47, 54, 56, 58, and 60. In addition, the nucleic acid may include a nucleic acid encoding a light chain variable region selected from the group consisting of SEQ ID NOS: 33, 37, 41, 45, and 49.

Specifically, the nucleic acid may include:
a nucleic acid of SEQ ID NO: 31 encoding a heavy chain variable region and a nucleic acid of SEQ ID NO: 33 encoding a light chain variable region;
a nucleic acid of SEQ ID NO: 35 encoding a heavy chain variable region and a nucleic acid of SEQ ID NO: 37 encoding a light chain variable region;
a nucleic acid of SEQ ID NO: 39 encoding a heavy chain variable region and a nucleic acid of SEQ ID NO: 41 encoding a light chain variable region;
a nucleic acid of SEQ ID NO: 43 encoding a heavy chain variable region and a nucleic acid of SEQ ID NO: 45 encoding a light chain variable region;
a nucleic acid of SEQ ID NO: 47 encoding a heavy chain variable region and a nucleic acid of SEQ ID NO: 49 encoding a light chain variable region;
a nucleic acid of SEQ ID NO: 54 encoding a heavy chain variable region and a nucleic acid of SEQ ID NO: 41 encoding a light chain variable region;
a nucleic acid of SEQ ID NO: 56 encoding a heavy chain variable region and a nucleic acid of SEQ ID NO: 41 encoding a light chain variable region;
a nucleic acid of SEQ ID NO: 58 encoding a heavy chain variable region and a nucleic acid of SEQ ID NO: 41 encoding a light chain variable region; or
a nucleic acid of SEQ ID NO: 60 encoding a heavy chain variable region and a nucleic acid of SEQ ID NO: 41 encoding a light chain variable region.

DNA encoding the antibody is readily separated or synthesized using conventional procedures (e.g., by using an oligonucleotide probe capable of specifically binding to DNA encoding heavy and light chains of the antibody) . Many vectors are available. Vector components generally include, but are not limited to, one or more of the following: signal sequences, origins of replication, one or more marker genes, enhancer elements, promoters, and transcription termination sequences.

The term "vector" as used herein is intended to include, as a means for expressing a target gene in a host cell, plasmid vectors, cosmid vectors, bacteriophage vectors, adenovirus vectors, retrovirus vectors, adeno-associated virus vectors, and the like. In the vector, the nucleic acid encoding the antibody is operatively linked to a promoter.

"Operatively linked" means a functional linkage between a nucleotide-expression-regulating sequence (for example, a promoter, a single sequence, or an array of transcription regulator-binding site) and other nucleotide sequences, and thus the nucleotide-expression-regulating sequence may regulate the transcription and/or translation of the other nucleotide sequences.

When a prokaryotic cell is used as a host, the vector generally includes a strong promoter capable of initiating transcription (e.g., a tac promoter, *lac* promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiating translation, and a transcription/translation termination sequence. In addition, for example, when a eukaryotic cell is used as the host, the vector may include a promoter derived from a mammalian genome (e.g., a metallothionein promoter, a β-actin promoter, a human hemoglobin promoter, and a human muscle creatine promoter) or a promoter derived from a mammalian virus (for example, an adenovirus late promoter, a vaccinia virus 7.5K promoter, a SV40 promoter, a cytomegalovirus (CMV) promoter, a *tk* promoter of HSV, a mouse breast tumor virus (MMTV) promoter, a HIV LTR promoter, a promoter of Moloney virus, an Epstein Barr virus (EBV) promoter, and a promoter of Rous sarcoma virus (RSV)), and generally has a polyadenylation sequence as a transcription termination sequence.

In some cases, the vector may be fused with other sequences to facilitate the purification of an antibody expressed therefrom. Fused sequences include, for example, glutathione S-transferase (Pharmacia, USA), maltose-binding protein (NEB, USA), FLAG (IBI, USA), and 6x His (hexahistidine; Qiagen, USA).

The vector includes an antibiotic resistance gene commonly used in the art as a selection marker, for example, resistance genes for ampicillin, gentamicin, carbenicillin, chloramphenicol, streptomycin, kanamycin, geneticin, neomycin, and tetracycline.

Another embodiment of the present disclosure relates to a cell transformed with the aforementioned vector. Cells used to produce the antibody of the present disclosure may be, but are not limited to, prokaryotic, yeast or higher eukaryotic cells.

Prokaryotic host cells such as *Escherichia coli,* strains of the genus *Bacillus,* such as *Bacillus subtilis* and *Bacillus thuringiensis, Streptomyces, Pseudomonas* (e.g., *Pseudomonas putida), Proteus mirabilis,* and *Staphylococcus* (e.g., *Staphylococcus carnosus)* may be used.

However, interest in animal cells is the greatest, and examples of useful host cell lines include COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080, but the present disclosure is not limited thereto.

Another embodiment of the present disclosure relates to a method of producing the antibody or antigen-binding fragment thereof, including: (a) culturing the cells; and (b) recovering the antibody or the antigen-binding fragment thereof from the cultured cells.

The cells may be cultured in various media. Any commercially available medium may be used as a culture medium without limitation. All other essential supplements known to those of ordinary skill in the art may also be included in suitable concentrations. Culture conditions, such as temperature and pH, have already been used with host cells selected for expression, which will be obvious to those of ordinary skill in the art.

The recovery of the antibody or antigen-binding fragment thereof may be performed by removing impurities by, for example, centrifugation or ultrafiltration, and purifying the result using, for example, affinity chromatography or the like. Other purification techniques, such as anion or cation exchange chromatography, hydrophobic interaction chromatography, hydroxylapatite chromatography, and the like, may be additionally used.

Another embodiment of the present disclosure relates to a composition for the prevention or treatment of tumors including the antibody as an active ingredient. The antibody may be a fragment including IgG or a variable region, namely ScFv or Fab. In addition, the variable region of a heavy chain may be IgG1, IgG2, IgG3, or IgG4.

The present disclosure provides a pharmaceutical composition for the prevention or treatment of eye diseases, including: (a) a pharmaceutically effective amount of an antibody against Ang2 or an antigen-binding fragment thereof according to the present disclosure; and (b) a pharmaceutically acceptable carrier. The present disclosure also provides a method of preventing or treating eye diseases, including administering the antibody or the antigen-binding fragment thereof to patients with tumors. The present disclosure also provides use of the antibody or the antigen-binding fragment thereof for inhibiting the mechanism of Ang2 and use thereof for the prevention or treatment of eye diseases.

With regard to eye diseases, the cornea is an avascular tissue, and must remain transparent at all times to preserve vision. However, it is known that angiogenesis also occurs in the eye, causing angiogenesis-related diseases of the eye. In other words, the formation of new blood vessels in the cornea reduces the transparency of the eye, causing loss of vision, and the creation of new blood vessels in the retina causes the formation of abnormal blood vessels, causing blood exudation to thereby induce blindness through the degeneration of retinal cells.

Based on this, the present disclosure may be used for the prevention or treatment of eye diseases such as premature retinopathy, corneal angiogenesis, diabetic retinopathy, choroidal neovascular disease, and macular degeneration (e.g., age-related macular degeneration).

The present disclosure provides a pharmaceutical composition for the prevention or treatment of tumors, including: (a) a pharmaceutically effective amount of an antibody against Ang2 or an antigen-binding fragment thereof according to the present disclosure; and (b) a pharmaceutically acceptable carrier. The present disclosure also provides a method of preventing or treating tumors, including administering the antibody or the antigen-binding fragment thereof to patients with tumors. The present disclosure also provides the use of the antibody or the antigen-binding fragment thereof for inhibiting the mechanism of Ang2 and the use thereof for the prevention or treatment of tumors.

Tumors, to which the composition is applicable, typically include tumors or cancers that overexpress Ang2, and tumors or cancers that do not overexpress Ang2. Nonlimiting examples of tumors or cancers that are suitable targets for treatment include melanoma (e.g., metastatic malignant melanoma), kidney cancer (e.g., clear-cell carcinoma), prostate cancer (e.g., hormone-refractory prostate adenocarcinoma), pancreatic adenocarcinoma, breast cancer (in some cases, triple-negative breast cancer), colon cancer, lung cancer (e.g., non-small-cell lung cancer), esophageal cancer, head and neck squamous cell carcinoma, liver cancer, ovarian cancer, cervical cancer, thyroid cancer, glioblastoma, glioma, leukemia, lymphoma, and other neoplastic carcinomas. Additionally, the present disclosure includes refractory or recurrent cancers that can be treated using the antibody of the present disclosure.

Another embodiment of the present disclosure relates to a pharmaceutical composition for inhibiting angiogenesis including the anti-Ang2 antibody or the antigen-binding fragment thereof as an active ingredient. Another embodiment provides a pharmaceutical composition for preventing and/or treating diseases related to angiopoietin-2 activation and/or overproduction including the anti-Ang2 antibody or the antigen-binding fragment thereof as an active ingredient.

The present disclosure provides, for example, a method of inhibiting angiogenesis, including administering a therapeutically effective amount of the anti-Ang2 antibody or the antigen-binding fragment thereof to a patient in need of angiogenesis inhibition. The method of inhibiting angiogenesis may further include identifying a patient in need of angiogenesis inhibition prior to administration. Another embodiment provides a method of preventing and/or treating diseases related to angiopoietin-2 activation and/or overproduction, including administering a therapeutically effective amount of the anti-Ang2 antibody or the antigen-binding fragment thereof to a patient in need of prevention and/or treatment of diseases related to angiopoietin-2 activation and/or overproduction. The method may further include, before administration, identifying a patient in need of the prevention and/or treatment of diseases related to angiopoietin-2 activation and/or overproduction.

The pharmaceutical composition may further include a pharmaceutically acceptable carrier, and the carrier may be one that is commonly used in formulating drugs, and may be one or more selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, but the present disclosure is not limited thereto. The pharmaceutical composition may further include one or more selected from the group consisting of diluents, excipients, lubricants, wetting agents, sweeteners, flavoring agents, emulsifiers, suspending agents, and preservatives, which are commonly used in the preparation of pharmaceutical compositions.

An effective amount of the pharmaceutical composition or the antibody or antigen-binding fragment thereof may be administered orally or parenterally. For parenteral administration, intravenous injection, subcutaneous injection, intramuscular injection, intraperitoneal injection, endothelial administration, topical administration, intranasal administration, intrapulmonary administration, intrarectal administration, or the like may be used. For oral administration, since a protein or a peptide is digested, an oral composition may be formulated such that active ingredients are coated or formulated to be protected from being decomposed in the stomach. In addition, the composition can be administered by any device that enables the active material to be delivered to a target cell.

The content of the anti-Ang2 antibody or antigen-binding fragment thereof in the pharmaceutical composition may be prescribed variously depending on factors such as formulation method, administration method, the age, body weight, and gender of a patient, pathologic conditions, diet, administration time, administration interval, administration route, excretion rate, and response sensitivity. For example, a daily dose of the anti-Ang2 antibody or the antigen-binding fragment thereof may range from 0.001 mg/kg to 1000 mg/kg, particularly 0.01 mg/kg to 100 mg/kg, and more particularly 0.1 mg/kg to 50 mg/kg, and further particularly 0.1 mg/kg to 20 mg/kg, but the present disclosure is not limited thereto. The daily dose may be formulated as a single dosage form in unit dose form, or may be formulated in appropriate portions, or may be prepared by being incorporated into a multiple-dose container.

The pharmaceutical composition may be administered in combination with other drugs, such as other angiogenesis inhibitors or therapeutic agents for diseases related to angiopoietin-2 activation and/or overproduction, and the administration amount and administration method thereof and the types of the other drugs may be appropriately prescribed depending on the condition of the patient.

The pharmaceutical composition may be formulated in the form of a solution in oil or an aqueous medium, a suspension, a syrup, an emulsion, an extract, a powder, granules, a tablet, a capsule, or the like, and may further include a dispersant or a stabilizer for formulation.

In particular, since the pharmaceutical composition including the anti-Ang2 antibody or the antigen-binding fragment thereof includes an antibody or an antigen-binding fragment, it may be formulated as an immune liposome. Liposomes including an antibody may be prepared according to a method well known in the art. The immune liposome is a lipid composition including phosphatidylcholine, cholesterol, and polyethylene glycol-derivatized phosphatidylethanolamine, and may be prepared by reverse-phase evaporation. (Publication Patent No. 10-2015-0089329). For example, a Fab' fragment of an antibody may be conjugated to liposomes through a disulfide replacement reaction.

Meanwhile, since the anti-Ang2 antibody or the antigen-binding fragment thereof specifically binds to angiopoietin-2, this characteristic can be used to confirm whether or not activation and/or overproduction of angiopoietin-2 occurs. Therefore, another embodiment of the present disclosure provides a pharmaceutical composition for the detection of angiopoietin-2 activation and/or overproduction, and/or for the diagnosis of diseases related to angiopoietin-2 activation and/or overproduction, including the anti-Ang2 antibody or the antigen-binding fragment thereof. Another embodiment provides a diagnosis method or a method of providing information on diagnosis, including: treating a biological sample obtained from a patient with the anti-Ang2 antibody or the antigen-binding fragment thereof; confirming whether or not an antigen-antibody reaction occurs; and determining that, when an antigen-antibody reaction is detected, the patient has a symptom of angiopoietin-2 activation and/or overproduction, or has a disease related to the activation and/or overproduction of angiopoietin-2. The biological sample may be selected from the group consisting of cells, tissues, and body fluids obtained from a patient.

The confirmation of whether or not an antigen-antibody reaction occurs may be performed through various methods known in the art. For example, reaction can be confirmed through conventional enzyme reaction, fluorescence, luminescence, and/or radiation detection, and particularly, may be measured using a method selected from the group consisting of immunochromatography, immunohistochemistry, enzyme-linked immunosorbent assay (ELISA), radioimmunoassay (RIA), enzyme immunoassay (EIA), fluorescence immunoassay (FIA), luminescence immunoassay (LIA), and western blotting, but the present disclosure is not limited thereto.

The patient to be administered or diagnosed with the pharmaceutical composition may be a mammal including primates including humans, monkeys, and the like, and rodents including mice, rats, and the like.

The disease related to angiopoietin-2 activation and/or overproduction may be cancer; metastatic cancer; eye diseases such as premature retinopathy, corneal angiogenesis, diabetic retinopathy, choroidal neovascular disease, and macular degeneration (e.g., age-related macular degeneration); asthma; rheumatoid arthritis; psoriasis; inflammatory diseases such as pneumonia and chronic inflammation; cardiovascular diseases such as hypertension or arteriosclerosis; or septicemia. The cancer may overexpress angiopoietin-2, may be solid cancer or blood cancer, and may be, but is not limited to, one or more selected from the group consisting of squamous cell carcinoma, small-cell lung cancer, non-small-cell lung cancer, adenocarcinoma of the lung, squamous cell carcinoma of the lung, peritoneal cancer, skin cancer, melanoma of the skin or eyes, rectal cancer, anal cancer, esophageal cancer, small-intestine cancer, endocrine adenocarcinoma, parathyroid cancer, adrenal cancer, soft-tissue sarcoma, urethral cancer, chronic or acute leukemia, lymphocytic lymphoma, hepatocellular carcinoma, gastrointestinal cancer, pancreatic cancer, glioblastoma, cervical cancer, ovarian cancer, liver cancer, bladder cancer, liver tumor, breast cancer (in some cases, triple-negative breast cancer), colon cancer, large intestine cancer, endometrial carcinoma or uterine carcinoma, salivary gland cancer, kidney cancer, liver cancer, prostate cancer, vulvar cancer, thyroid cancer, liver cancer, head and neck cancer, brain cancer, and osteosarcoma. The cancer may be primary cancer or metastatic cancer.

### Examples

Hereinafter, the present disclosure will be described in further detail with reference to the following examples. These examples are provided for illustrative purposes only, and it will be obvious to those of ordinary skill in the art that these examples should not be construed as limiting the scope of the present disclosure.

### Example 1. Selection of Antibodies Binding to Ang2

For the preparation of antibody libraries and libraries for selecting antibodies that bind to Ang2, the human sensitized scFv (human naive ScFv) library as used in Korean Patent Application (Patent Publication No. 10-2008-0109417) is used. 2 µg/ml (100 µl per well) of an antigen (hAng2-his: RND systems. Cat. No 623-AN/CF, hAng2-Fc: PharmAbcine) is added to a 96-well immunoplate and allowed to stand overnight at 4 °C. The next day, the antigen-coated plate is washed 3 times with 5 mM CaCl₂ TBS, and then 200 µl of 2% BSA blocking buffer is added and allowed to react at room temperature for 2 hours. 50 µl of a XL1-Blue stock is added to 2 ml of a 2x YT-TET (tetracycline 10 µg/ml) growth medium and grown at 37° C and 200 rpm for about 2 hours, and then 13 ml thereof is further added to grow the stock until OD₆₀₀ reached 0.5. After 2 hours of blocking, the resultant is washed three times with 1X 5 mM CaCl₂ TBS. The phage library group is combined with each washed well, and the phage library and 4% BSA are mixed in the same amount, and then 200 µl of the mixture is added to each well, followed by rocking at room temperature for 30 minutes and reaction for 2 hours. When the phage library reaction is completed, the supernatant is discarded, the resulting solution is washed 5 times with 0.1% TBST (5mM CaCl₂) and 5 times with TBS (5mM CaCl₂), and 100 µl of 100 mM trimethylamine (TEA) is added to each well, followed by shaking at room temperature for 10 minutes. After 10 minutes, 50 µl of 1 M Tris (pH 7.5) is added to each well and mixed. The supernatant is added to 10 ml of XL1-blue with OD₆₀₀ of 0.5 for infection at 37 °C for 30 minutes. After infection, 100 µl is used as an output titer, and the remainder is centrifuged at 6,000 rpm for 10 minutes. The supernatant is discarded, and the precipitate is spread over a large square plate (34 µg/ml of CM + 1% glucose) and incubated overnight at 30 °C. 100 µl remaining as an output titer is diluted to 1/10, 1/100, or 1/1000, spread over a CM plate, and allowed to stand overnight at 37 °C. The next day, colonies grown on the square plate are put in 50 ml of a 2x YT medium, scraped using a loop, and then centrifuged at 6000 rpm for 10 minutes to discard the supernatant, and the precipitate is prepared into a primary panning stock, 100 ml of a 2x YT growth medium (34 µg/ml of CM + 1% Glucose) is put in a 500 ml Erlenmeyer flask, and then cells are added thereto so that OD₆₀₀ becomes 0.2 and allowed to grow at 200 rpm and 37 °C until OD₆₀₀ becomes 0.5. After cells are cultured until the OD₆₀₀ value becomes 0.5, a helper phage (M13K07 mutant) is added in an amount of 20 times greater than OD₆₀₀ value of the cells. After the helper phage is added and infection is allowed to occur at 37 °C for 30 minutes, centrifugation is performed at 6000 rpm for 10 minutes. The supernatant is discarded, and 100 ml of a 2xYT medium (34 µg/ml of CM + 70 µg/ml of Kan. + 1 mM IPTG + 5 mM MgCl₂) is added to the cells, which are then allowed to stand overnight at 200 rpm and 30 °C. The next day, the grown cells are centrifuged at 7000 rpm for 10 minutes and centrifuged once again using the same method. 20% PEG/2.5 m NaCl is added to 1/5 (v/v) of the supernatant from the collected supernatant and precipitated on ice for 1 hour. After precipitation, centrifugation is performed at 9000 rpm for 1 hour. The supernatant is discarded, and the precipitate is released with 3 ml of TBS, filtered with a 0.45 µm filter, and stored at 4 °C for use in a subsequent panning process. These processes are repeated three times to four times, and antibodies binding to an antigen were identified by ELISA.

### Example 2. Screening of Monoclonal ScFv phage that Specifically Binds to Ang2 and Neutralizes binding with Tie2 (binding ELISA/competitive ELISA)

After the panning process is completed, the final round cell stock is diluted and spread so that 200 to 500 colonies can be formed on a CM agar plate, and is then allowed to stand overnight at 37 °C. The next day, when the colonies grow, 200 µl of 2xYT medium (34 µg/ml of CM + 1% glucose) was added to a 96-well deep plate and the colonies are added to each well one by one and allowed to stand overnight at 37 °C and 3000 rpm. The next day, 200 µl of 2xYT medium (34 µg/ml of CM + 1% glucose) is added to a new 96-well deep plate and 20 ml of the cells grown on the previous day are added to each well and then grown at 37 °C and 3000 rpm for 1 hour and 10 minutes. 100 µl of 50% glycerol is added to each well, and the remaining cells are stored at -70 °C. When the cells are grown, 1 µl of a helper phage and 19 µl of 2xYT medium are mixed, and then 20 µl of the resultant mixture is added to each well, followed by incubation at 37 °C for 30 minutes. After the incubation is completed, centrifugation is performed at 3000 rpm for 10 minutes. The supernatant is discarded and 200 µl of 2xYT medium (34 µg/ml of CM + Kan. 70 µg/ml + 1 mM IPTG + 5 mM MgCl₂) is added and allowed to stand overnight in Megagrow at 30 °C and 3000 rpm.

In order to select phages that specifically bind to Ang2, 1 µg/ml (100 µl/well) of Ag (hAng2-Fc, hAng1-his: RND systems. Cat. No 923-AN/CF or mAng1-Fc, PharmAbcine) is added to a 96-well immunoplate and allowed to stand overnight at 4 °C. The next day, the cells grown on the previous day were centrifuged at 3000 rpm for 10 minutes and stored at 4 °C. The spread Ag is washed three times with 0.1% TBST (5 mM CaCl₂), and then 200 µl of 2% BSA blocking buffer is added, followed by incubation at 25 °C for 2 hours. After blocking is completed, washing is performed three times with 0.1% TBST (5 mM CaCl₂). 50 µl of 4% BSA and 50 µl of the phage spun down and stored at 4 °C are mixed in each well, and shaken at room temperature for 1 hour to allow a reaction to occur. After phage binding, washing is performed three times with 0.1% TBST (5 mM CaCl₂), and then 100 µl of HRP-conjugated mouse anti-M13 Ab 1:3000 (Sino, 11973-MM05) is added and a reaction is allowed to occur at 25 °C for 1 hour. After the reaction is completed, washing is performed three times with 0.1% TBST (5 mM CaCl₂), 100 µl of TMB (#BD TMB substrate reagent set 555214) is added to develop color for 3-5 minutes, and then 50 µl of a stop solution is added to each well, followed by analysis using an ELISA reader.

**[Table 1] ELISA measurement results of monoclonal scFv phage specifically binding to Ang2 antigen**

| Clone | OD | | |
|---|---|---|---|
| Antigen | hAng2-Fc | hAng1-his | mAng1-Fc |
| No. 3 | 1.904 | 0.025 | - |
| No. 4 | 0.835 | - | 0.093 |
| No. 8 | 1.364 | - | 0.199 |
| No. 41 | 0.825 | - | 0.111 |
| No. 46 | 1.194 | - | 0.061 |

The base sequences of selected antibodies are shown in Tables 2 and 3 below.

**[Table 2] CDR sequences of antibodies specifically binding to Ang2 antigen**

| Sequence name | | Amino acid sequence | SEQ ID NO: |
|---|---|---|---|
| No.3 | Heavy-chain CDR1 | GFSFDDYA | 1 |
| | Heavy-chain CDR2 | IKDDGSQT | 2 |
| | Heavy-chain CDR3 | TTEGLMNGLHFDM | 3 |
| | Light-chain CDR1 | SSNIGAGYD | 4 |
| | Light-chain CDR2 | GNN | 5 |
| | Light-chain CDR3 | QSYDSRLGW | 6 |
| No.4 | Heavy-chain CDR1 | GYSFTSYW | 7 |
| | Heavy-chain CDR2 | IYPGNSDT | 8 |
| | Heavy-chain CDR3 | TTEGLMNGLHFDM | 9 |
| | Light-chain CDR1 | QSLLHSLGDNY | 10 |
| | Light-chain CDR2 | LGS | 11 |
| | Light-chain CDR3 | MQSLQTPPYT | 12 |
| No.8 | Heavy-chain CDR1 | GFTFSSYS | 13 |
| | Heavy-chain CDR2 | ISASDGAT | 14 |
| | Heavy-chain CDR3 | AKILAGYSGPMGGMDV | 15 |
| | Light-chain CDR1 | RDISNY | 16 |
| | Light-chain CDR2 | GAS | 17 |
| | Light-chain CDR3 | QQYYSYPLT | 18 |
| No.41 | Heavy-chain CDR1 | GFAFGRYE | 19 |
| | Heavy-chain CDR2 | IDTGGGAK | 20 |
| | Heavy-chain CDR3 | TTEGLMNGLHFDM | 21 |
| | Light-chain CDR1 | QAISTW | 22 |
| | Light-chain CDR2 | TAS | 23 |
| | Light-chain CDR3 | QQLNSYPYT | 24 |
| No.46 | Heavy-chain CDR1 | GFTFDDCA | 25 |
| | Heavy-chain CDR2 | ISGNSKNV | 26 |
| | Heavy-chain CDR3 | ARDPAYSQFDY | 27 |
| | Light-chain CDR1 | SSNVGGYP | 28 |
| | Light-chain CDR2 | TDY | 29 |
| | Light-chain CDR3 | ATWDDNLNGYV | 30 |

**[Table 3] Variable region sequences of antibodies specifically binding to Ang2 antigen**

| Antibod y name | | Sequence | SEQ ID NO: |
|---|---|---|---|
| No.3 | Heavy chain variable region | CAGATGCAGCTGGTGCAGTCTGGGGGAGG CTTGGTACAGCCTGGCAGGTCCCTCAAAC TCTCCTGCGCAGCCTCTGGATTCTCCTTT GATGATTATGCCATGCACTGGGTCCGGCA AGCTCCAGGGAAGGGGCTGGAGTGGGTGG CCACCATAAAGGACGATGGAAGTCAGACA TACTATGTGGACTCTGTGAAGGGCCGATT CACCATCTCCAGAGACAACGCCAAGAGCT CACTGTTTCTGCAAATGAACAGTCTGAGA GCCGAGGACACGGCCGTGTATTACTGTAC | 31 |
| | | CACAGAAGGATTAATGAATGGACTTCATT TTGATATGTGGGGCCAAGGGACAATGGTC ACCGTCTCCTCA | |
| | | CACAGAAGGATTAATGAATGGACTTCATT TTGATATGTGGGGCCAAGGGACAATGGTC ACCGTCTCCTCA QMQLVQSGGGLVQPGRSLKLSCAASGFSF DDYAMHWVRQAPGKGLEWVATIKDDGSQT YYVDSVKGRFTISRDNAKSSLFLQMNSLR AEDTAVYYCTTEGLMNGLHFDMWGQGTMV TVSS | 32 |
| | Light chain variable region | CAGCTCGTGCTGACTCAGCCGCCCTCAGT GTCTGGGGCCCCAGGGCAGGGGGTCACCA TCTCCTGCACTGGGAGCAGCTCCAACATC GGGGCAGGTTATGATGTACACTGGTACCA GCAGTTTCCAGGAACAGCCCCCAAACTCC TCATCTCTGGTAACAATAATCGGCCCTCA GGGCTCCCTGACCGATTCTCTGGCTCCAA GTCTGGCACCTCAGCCTCCCTGGCCATCA CTGGACTCCAGGCTGAGGATGAGGCTGAT TATTACTGCCAGTCCTATGACAGCAGGCT GGGTGTGGTCTTCGGCGGAGGGACCAAGC TGACCGTCCTAGGT LVLTQPPSVSGAPGQGVTISCTGSSSNIG AGYDVHWYQQFPGTAPKLLISGNNNRPSG LPDRFSGSKSGTSASLAITGLQAEDEADY YCQSYDSRLGVVFGGGTKLTVLG | 33 |
| | | LVLTQPPSVSGAPGQGVTISCTGSSSNIG AGYDVHWYQQFPGTAPKLLISGNNNRPSG LPDRFSGSKSGTSASLAITGLQAEDEADY YCQSYDSRLGVVFGGGTKLTVLG | 34 |
| No.4 | Heavy chain variable region | CAGGTGCAGCTGGTGGAGTCTGGAGCAGA GGTGAAAAGACCCGGGGAGTCTCTGAGGA TCTCCTGTAAGACTTCTGGATACAGCTTT ACCAGCTACTGGATCCACTGGGTGCGCCA GATGCCCGGGAAAGAACTGGAGTGGATGG GGAGCATCTATCCTGGGAACTCTGATACC AGATACAGCCCATCCTTCCAAGGCCACGT CACCATCTCAGCCGACAGCTCCAGCAGCA CCGCCTACCTGCAGTGGAGCAGCCTGAAG GCCTCGGACACCGCCATGTATTACTGTAC CACAGAAGGATTAATGAATGGACTTCATT TTGATATGTGGGGCCAAGGGACAATGGTC ACCGTCTCCTCA QVQLVESGAEVKRPGESLRISCKTSGYSF TSYWIHWVRQMPGKELEWMGSIYPGNSDT RYSPSFQGHVTISADSSSSTAYLQWSSLK ASDTAMYYCTTEGLMNGLHFDMWGQGTMV TVSS | 35 |
| | | QVQLVESGAEVKRPGESLRISCKTSGYSF TSYWIHWVRQMPGKELEWMGSIYPGNSDT RYSPSFQGHVTISADSSSSTAYLQWSSLK ASDTAMYYCTTEGLMNGLHFDMWGQGTMV TVSS | 36 |
| | Light chain variable region | GATATTGTGATGACCCAGACTCCACTCTC CCTGCCCGTCACCCCTGGAGAGCCGGCCT CCATCTCCTGTAGGTCAAGTCAGAGCCTC CTGCATAGTCTTGGAGACAATTATTTGGA TTGGTATCTACAGAAGCCAGGGCAGTCTC CGCAACTCCTGATCTATTTGGGTTCTAAG CGGGCCGCCGGGGTCCCCGACAGGTTCAG TGGCAGTGGCTCAGGCACAGACTTTACAC TCAAAATCAGCAGAGTGGAGGCTGAGGAT GTTGGAGTTTATTATTGCATGCAATCTCT | 37 |
| | | ACAAACTCCCCCGTACACTTTTGGCCAGG GGACCAAGCTGGAGATCAAACGT | |
| | | ACAAACTCCCCCGTACACTTTTGGCCAGG GGACCAAGCTGGAGATCAAACGT DIVMTQTPLSLPVTPGEPASISCRSSQSL LHSLGDNYLDWYLQKPGQSPQLLIYLGSK RAAGVPDRFSGSGSGTDFTLKISRVEAED VGVYYCMQSLQTPPYTFGQGTKLEIKR | 38 |
| No.8 | Heavy chain variable region | CAGGTGCAGCTGGTAGAGTCTGGGGGAGG CCTGGTCAAGCCTGGGGGGTCCCTGAGAC TCTCCTGTGCAGCCTCTGGATTCACCTTC AGTAGCTATAGCATGAACTGGGTCCGCCA GGCTCCAGGGAAGGGGCTGGAGTGGGTCT CATCCATTAGTGCTAGTGATGGTGCCACA TACTACGCAGACTCCGTGAGGGGCCGGTT CACCATCTCCAGAGACAATTCCAGGAGCA CACTGTATCTGCAAATGAACAGTCTGAGA GCCGAGGACACGGCCGTGTATTACTGTGC GAAAATTCTCGCGGGATATAGTGGCCCAA TGGGCGGAATGGACGTCTGGGGCCAAGGG ACCACGGTCACCGTCTCCTCA QVQLVESGGGLVKPGGSLRLSCAASGFTF SSYSMNWVRQAPGKGLEWVSSISASDGAT YYADSVRGRFTISRDNSRSTLYLQMNSLR AEDTAVYYCAKILAGYSGPMGGMDVWGQG TTVTVSS | 39 |
| | | QVQLVESGGGLVKPGGSLRLSCAASGFTF SSYSMNWVRQAPGKGLEWVSSISASDGAT YYADSVRGRFTISRDNSRSTLYLQMNSLR AEDTAVYYCAKILAGYSGPMGGMDVWGQG TTVTVSS | 40 |
| | Light chain variable region | GACATCCAGATGACCCAGTCTCCATCCTC ACTGTCTGCATCTGTAGGAGACAGAGTCA CCATCACTTGTCGGGCGAGTCGGGACATT AGCAACTATTTAGCCTGGTATCAGCAGAA ACCAGGGAAAGCCCCTAAGTCCCTGATCT ATGGAGCATCCAATTTACAAAGTGGGGTC TCATCACAGTTCAGCGGCAGTGGATCCGG GACAGATTTCACCCTCACCATCAACAGCC TGCAGCCTGAAGATTCTGCAACTTATTAC TGTCAACAGTACTATAGTTACCCGCTCAC TTTTGGCGGAGGGACCAAGGTGGATATCA AACGT DIQMTQSPSSLSASVGDRVTITCRASRDI SNYLAWYQQKPGKAPKSLIYGASNLQSGV SSQFSGSGSGTDFTLTINSLQPEDSATYY CQQYYSYPLTFGGGTKVDIKR | 41 |
| | | DIQMTQSPSSLSASVGDRVTITCRASRDI SNYLAWYQQKPGKAPKSLIYGASNLQSGV SSQFSGSGSGTDFTLTINSLQPEDSATYY CQQYYSYPLTFGGGTKVDIKR | 42 |
| No.41 | Heavy chain variable region | CAGGTGCAGCTGGTGGAGTCTGGGGGAGG CTTGGTACAGCCTGGAGGGTCCCTGAGAC TCTCCTGTGAAGCCTCTGGATTCGCCTTC GGTCGTTATGAGATGAATTGGGTCCGCCA GGCTCCAGGGAAGGGGCTGGAGTGGATTG CATATATTGATACTGGTGGTGGTGCCAAA GTCTATGCAGACTCTGTGAAGGGCCGATT CACCATCTCCAGAGACGACAGCAAAAACT CCCTGTATCTGCAAATGAACAGTCTGAGA GCCGAGGACACGGCCGTGTATTACTGTAC CACAGAAGGATTAATGAATGGACTTCATT | 43 |
| | | TTGATATGTGGGGCCAAGGGACAATGATC ACCGTCTCCTCA | |
| | | QVQLVESGGGLVQPGGSLRLSCEASGFAF GRYEMNWVRQAPGKGLEWIAYIDTGGGAK VYADSVKGRFTISRDDSKNSLYLQMNSLR AEDTAVYYCTTEGLMNGLHFDMWGQGTMI TVSS | 44 |
| | Light chain variable region | GACATCCAGATGACCCAGTCTCCTTCCAC CCTGTCTGCATCTGTAGGAGACAGAGTCG CCATCACTTGCCGGGCCAGTCAGGCTATT AGTACCTGGTTGGCCTGGTATCAGCAGAA ACCTGGTAAAGCCCCTAAACTCCTGATCT ATACGGCGTCTACTTTAGAAAGTGGGGTC CCATCAAGGTTCAGCGGCAGTGGATCTGG GACAGATTTCACTCTCACCATCAACAGCC TGCAGCCTGATGATTTTGCAACTTATTAC TGTCAACAGCTTAATAGTTACCCTTACAC TTTCGGCGGAGGGACCAAGGTGGAGATCA AACGT | 45 |
| | | DIQMTQSPSTLSASVGDRVAITCRASQAI STWLAWYQQKPGKAPKLLIYTASTLESGV PSRFSGSGSGTDFTLTINSLQPDDFATYY CQQLNSYPYTFGGGTKVEIKR | 46 |
| No.46 | Heavy chain variable region | CAGGTGCAGCTGGTGGAGTCTGGGGGAGG CTTGGTACAGCCTGGCAGGTCCCTGAGAC TCTCCTGTGCAGCCTCTGGATTCACCTTT GATGATTGTGCCATGCACTGGGTCCGACA AGCTCCAGGGAAGGGCCTGGAGTGGGTCT CAGGTATTAGTGGGAATAGTAAAAACGTA GCCTATGCGGACTCTGTGAAGGGCCGATT CAGCATCTCCAGAGACGACGCCAAGAACT CCCTGTATCTGCAAATGAACAGTCTGAGA GCCGAGGACACGGCCGTGTATTACTGTGC GAGAGATCCGGCATACAGCCAGTTTGACT ACTGGGGCCAGGGAACCCTGATCACCGTC TCCTCA | 47 |
| | | QVQLVESGGGLVQPGRSLRLSCAASGFTF DDCAMHWVRQAPGKGLEWVSGISGNSKNV AYADSVKGRFSISRDDAKNSLYLQMNSLR AEDTAVYYCARDPAYSQFDYWGQGTLITV SS | 48 |
| | Light chain variable region | CAGTCTGCCCTGACTCAGCCTCCCTCACT GTCTGCGACCCCCGGGCAGAGGGTCACCA TCTCTTGCTCTGGAAGCAGCTCCAACGTC GGAGGATATCCTGTCAACTGGTACCAGCA GGTCCCAGGAGCGGCCCCCAAACTCCTCA TGTATACTGATTATAAGCGGCCCTCAGGT GTCCCTGACCGATTCTTTGGCTCCAAGTC TGGCACTTCAGCCTCCCTGGCCATCAGTG GCCTCCAGTCTGAAGATGAGGCTGATTAT TACTGTGCTACATGGGACGACAACCTGAA | 49 |
| | | TGGCTATGTCTTCGGAACTGGGACCAAGG TCACCGTCCTAGGT | |
| | | QSALTQPPSLSATPGQRVTISCSGSSSNV GGYPVNWYQQVPGAAPKLLMYTDYKRPSG VPDRFFGSKSGTSASLAISGLQSEDEADY YCATWDDNLNGYVFGTGTKVTVLG | 50 |

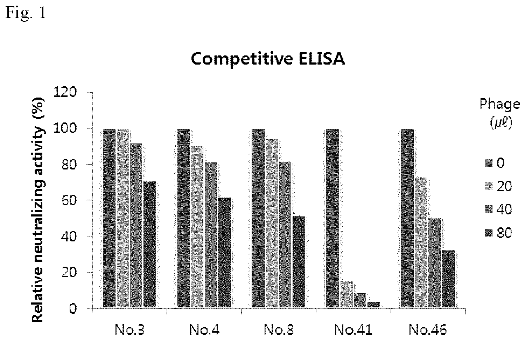

To select phages that neutralize the binding of Ang2/Tie2 in competitive ELISA, 1 µg/ml (100 µl per well) of Ag (hTie2-Fc: PharmAbcine) is added to a 96-well immunoplate and allowed to stand overnight at 4 °C. The spread Ag is washed twice with 1X PBS, and then 200 µl of 3% BSA blocking buffer is added, followed by incubation at 25 °C for 2 hours. After blocking is completed, washing is performed twice with 0.1% PBST. In each well, 20 µl (5 µg/ml) of hAng2-his (RND, 623-AN/CF) is mixed with the phage, which has been cooled down and stored at 4 °C, according to various volumes (80 µl, 40 µl+1X PBS 40 µl, 20 µl+1X PBS 60 µl), and shaken at room temperature for 1 hour to allow a reaction to occur. After phage binding, washing is performed three times with 0.05% PBST, followed by reaction at room temperature for 1 hour with 0.5 µg/ml of anti-Ang2 mouse antibodies. After antibody binding is completed, washing is performed three times with 0.05% PBST, and then 100 µl of HRP-conjugated mouse anti-IgG Ab 1:2000 (RND, HAF007) is added and a reaction is allowed to occur at 25 °C for 1 hour. After the reaction is completed, washing is performed three times with 0.05% PBST, 100 µl of TMB (#BD TMB substrate reagent set 555214) is added to develop color for 3-5 minutes, and then 50 µl of a stop solution is added to each well, followed by analysis using an ELISA reader. The results thereof are illustrated in FIG. 1. As illustrated in FIG. 1, it was confirmed that the selected phages had the ability to neutralize the binding of Tie2/Ang2.

### Example 3. Selection of Antibodies with High Affinity for Ang2 (Off-rate screening)

The binding affinity of the selected antibodies to the antigen was measured using Octet (Fortebio). To this end, Ang2 was immobilized on a biosensor, and then candidate antibodies expressed in the form of scFv were added and allowed to bind thereto, followed by measurement of dissociation rate constants. The results thereof are shown in Table 4.

**[Table 4] Dissociation rate constants of antibodies specifically binding to Ang2 antigen**

| Clone | Kdis (1/s) |
|---|---|
| No.3 | 6.42E-04 |
| No.4 | 2.12E-04 |
| No. 8 | <1.0E-07 |
| No.41 | 1.08E-05 |
| No.46 | 6.14E-05 |

### Example 4. Anti-Ang2 Antibody Expression

Conversion of the selected scFv phages into IgG form was carried out using a molecular biological technique. Phagemid was extracted from the selected *E. coli* clones, and the variable regions were amplified using a PCR technique. The amplified heavy chain variable region was inserted into an expression vector (Invivogen, pfusess-hchg1) containing a heavy chain constant region, and the amplified light chain variable region was inserted into an expression vector (Invivogen, pfuse2ss-hclk) containing a light chain constant region, thereby completing the cloning of IgG-type DNA.

The transient expression of IgG was performed using an Expi293F expression system kit (Thermo Fisher Scientific, US). Expi293 cells included in the kit were suspended and incubated on a 125 rpm orbital shaker at 37 °C and in a 5% CO₂ environment using an exclusive medium. Every three days, the cells were passaged to a concentration of 3 × 10⁵ cells/ml, and when introduced into an expression vector, the number of the cells was adjusted to 3 × 10⁶ cells/ml before use. For gene introduction, Expifectamine, which is an exclusive reagent, was used, and a lipid-DNA complex containing 1 µg of expression vector DNA and 2.7 µl of Expifectamine per 1 ml of a cell suspension was produced and added to the cell suspension, and 16-18 hours after introduction, enhancer 1/2 was added to induce expression. Thereafter, the resultant suspension was cultured for 3-4 days under the same conditions and then centrifuged to collect an IgG-containing supernatant.

### Example 5. Purification of Anti-Ang2 Antibody

The collected supernatant was injected into a Protein A column (GE Healthcare) to purify IgG through affinity chromatography. The column was equilibrated with 20 mM Tris-HCl, 50 mM NaCl, and 5 mM EDTA (pH 7.0), and then the supernatant was injected, washing was performed using 50 mM Tris-HCl, 500 mM NaCl, 5 mM EDTA, 0.2% polysorbate 20 (pH 7.0), and then elution was performed with 50 mM NaCl, 0.1 M glycine-HCl (pH 3.5), followed by neutralization with 1 M Tris. For the eluted proteins, the solvent was replaced with PBS through dialysis using a MWCO 10,000 spectra/por dialysis membrane (Spectrum Labs, US). Thereafter, the proteins were concentrated using Vivaspin (Satorius, DE) to a required concentration, dispensed, and then stored at -80 °C.

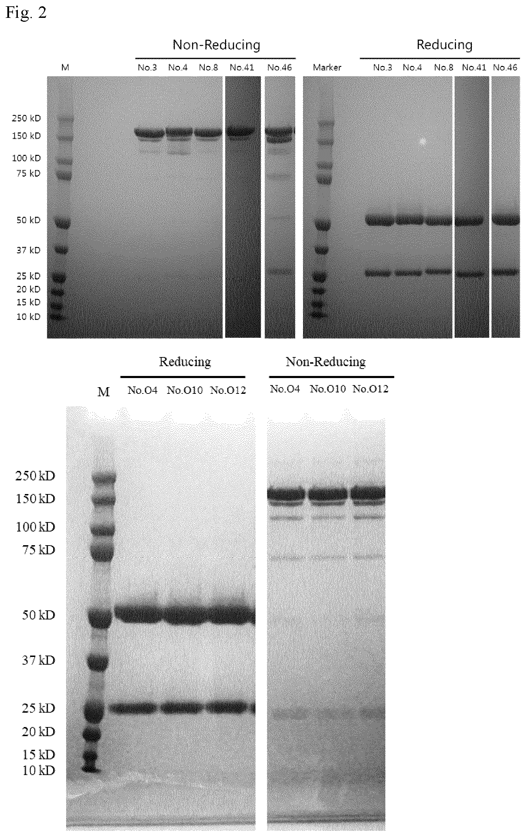

After purification, each antibody was treated in nonreducing and reducing LDS sample buffer (Thermo Fisher Scientific) and electrophoresed using a NuPAGE System (Thermo Fisher Scientific). As a result, IgG including a 50 kDa heavy chain and a 25 kDa light chain and having a total molecular weight of about 150 kDa was obtained (FIG. 2).

### Example 6. Analysis of Binding Specificity of Anti-Ang2 Antibody

For binding specificity analysis, binding constants were measured using an ELISA method and a Biacore T200 system (GE Healthcare Life Sciences).

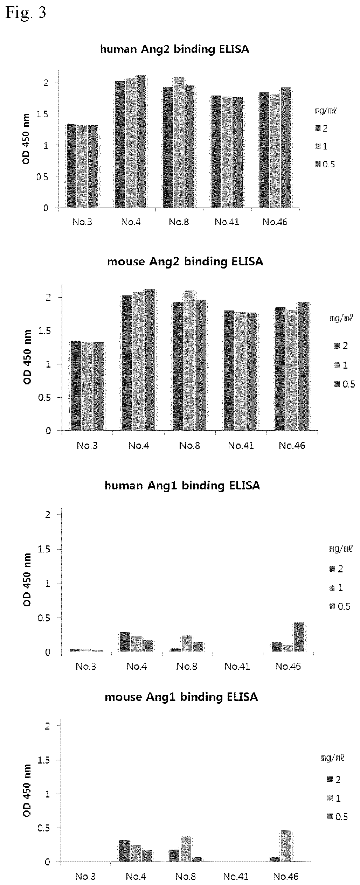

1 ug/ml (100 µl per well) of a His-tagged human (R&D systems, 623-AN/CF) or mouse (sino, 50298-M07H) Ang2 solution or a His-tagged human (R&D systems, 923-AN/CF) or mouse (sino, 50300-M07H) Ang1 solution was added to a 96-well immunoplate (Nunc, US) and allowed to stand overnight at 4 °C for adsorption. The next day, the solution was washed three times with PBS containing 0.05% Tween-20 (hereinafter, referred to as PBST), and 200 µl of a 2% BSA/PBST solution was added to each well and then allowed to stand at room temperature for 2 hours to perform blocking. After washing three times with PBST, 100 µl of each test antibody solution was added to each well according to concentration to cause binding at room temperature for 1 hour, and then washing was performed three times with PBST, 100 µl of HRP-conjugated goat anti-human IgG(kappa) (bethyl lab #A80-115P) diluted to 1:2000 was added and allowed to react at room temperature for 1 hour, thereby inducing binding, and after washing three times with PBST, color development was performed using 100 µl of a TMB substrate reagent. The color development reaction was stopped by adding 50 µl of 2N H₂SO₄, and a Sunrise microplate reader (TECAN, CH) was used to measure specific absorbance OD₄₅₀₋₆₃₀ (FIG. 3). As illustrated in FIG. 3, the selected antibodies specifically bind to human and mouse Ang2 and do not bind to human and mouse Ang1.

To analyze the binding affinity of the selected anti-Ang2 antibodies, affinity analysis for human Ang2 and mouse Ang2 was performed using BIACORE^{®} T200 (GE Healthcare). A protein A sensor chip was used, and an experiment was conducted in accordance with the manufacturer's manual. Detailed analysis conditions are as follows. 2000 response units (RU) of protein A were immobilized, 25 RU of anti-Ang2 antibody candidates were used for binding, and various concentrations of human Ang2 and mouse Ang2 were allowed to bind. The starting concentrations for analysis are 100 nM and 150 nM, respectively. The analysis proceeded at a flow rate of 30 µl/min, the binding and dissociation time of human Ang2 was measured as 300 seconds and 2000 seconds, respectively, and the binding and dissociation time of mouse Ang2 was analyzed as 300 seconds and 1000 seconds, respectively. Analysis was performed using a 1:1 binding model as an analytical model. The analysis results thereof are shown in Table 5.

**[Table 5]**

| | SPR (Biacore T-200) | | | | | |
|---|---|---|---|---|---|---|
| | hAng2 | | | mAng2 | | |
| Sample | ka (1/Ms) | kd(1/s) | KD (pM) | ka (1/Ms) | kd(1/s) | KD (pM) |
| No.3 | 3.34E+05 | 4.22E-05 | 127 | 1.58E+05 | 8.53E-05 | 538 |
| No.4 | 2.73E+05 | 5.93E-05 | 217 | 1.57E+05 | 7.05E-05 | 450 |
| No.8 | 2.68E+05 | 4.37E-05 | 163 | 1.44E+05 | 6.69E-05 | 463 |
| No.41 | 3.20E+05 | 7.77E-05 | 243 | 2.89E+05 | 9.47E-05 | 328 |
| No.46 | 2.74E+05 | 8.62E-05 | 314 | 4.65E+05 | 8.94E-05 | 193 |
| nesvacumab | 3.53E+05 | 9.30E-05 | 263 | 2.10E+05 | 5.51E-05 | 262 |

### Example 7. Confirmation of Neutralization Ability of Anti-Ang2 Antibodies (Ang2/Tie2, Ang2/integrin)

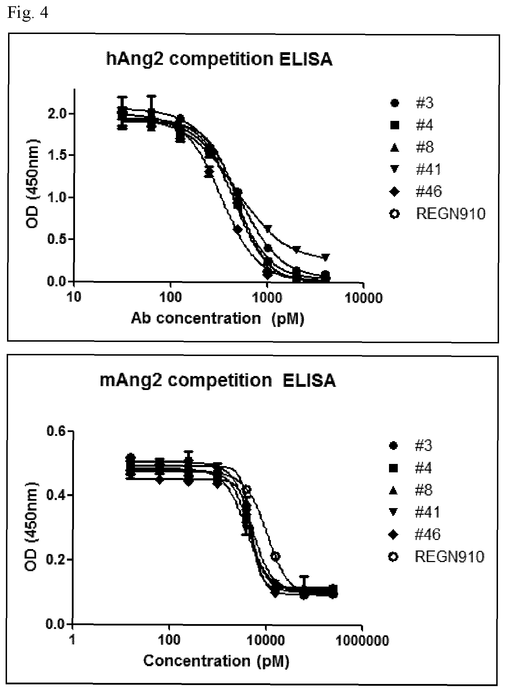

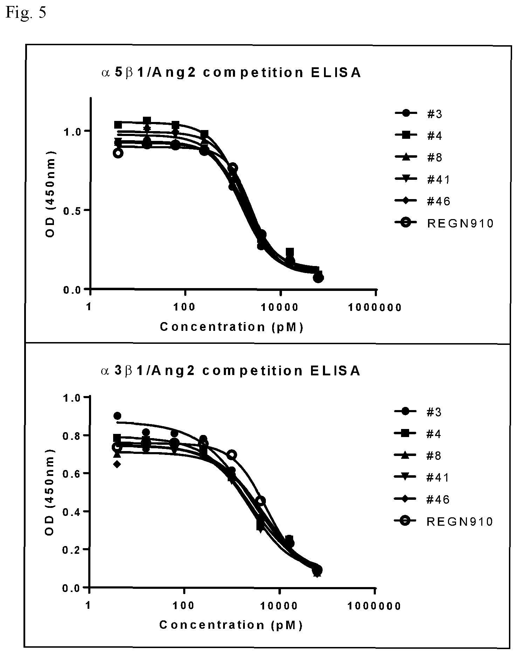

1 ug/ml (100 µl per well) of a human Ti32-Fc (R&D Systems, 313-TI) or mouse Tie2-Fc (R&D Systems, 762-T2-100) solution, an integrin α3/β1 solution (R&D systems, 2840-A3-050), or an integrin α5/β1 solution (R&D systems, 3230-A5-050) was added to a 96-well immunoplate (Nunc, US) and then allowed to stand overnight at 4 °C for adsorption. The next day, the plate was washed four times with PBS containing 0.05% Tween-20 (hereinafter, referred to as PBST), and 200 µl of a 2% BSA/PBST solution was added to each well and then allowed to stand at room temperature for 1 hour to perform blocking. Anti-Ang2 candidate antibodies were serially diluted 4-fold at a maximum of 1000 nM, and biotinylated human Ang2 protein was made to a final concentration of 100 ng/mL or 625 ng/mL, followed by previous binding at room temperature for 1 hour. The plate, which had undergone blocking, was washed four times with PBST, and then samples in which the antigen-antibody reaction was previously induced were added to the plate, and binding was allowed to occur at room temperature for 1 hour. The plate was washed three times with PBST, 100 µl of HRP-conjugated streptavidin (R&D Systems, DY998) diluted to 1:200 was added to allow a reaction to occur at room temperature for 1 hour, thereby inducing binding, and the plate was washed four times with PBST, followed by color development using 100 µl of a TMB substrate reagent. The color development reaction was stopped by adding 50 µl of 2N H₂SO₄, and a Sunrise microplate reader (TECAN, CH) was used to measure specific absorbance OD₄₅₀ (FIGS. 4 and 5). As illustrated in FIG. 4, the selected antibodies neutralized Ang2/Tie2 binding both in humans and mice. The neutralization capacity was quantified by obtaining the IC₅₀ value and is shown in Table 6. In addition, as illustrated in FIG. 5, the selected antibodies also neutralized integrin/Ang2 binding. The neutralization ability of integrin/Ang2 was quantified using the IC₅₀ value, and the results thereof are shown in Table 7.

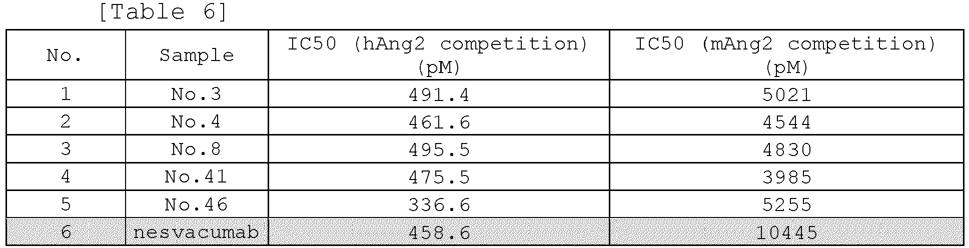

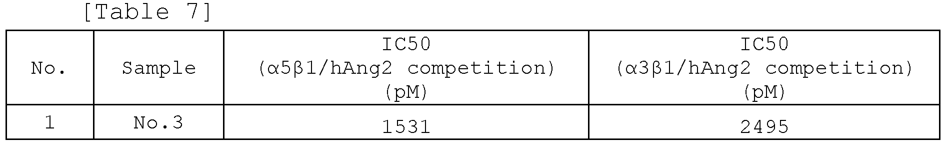

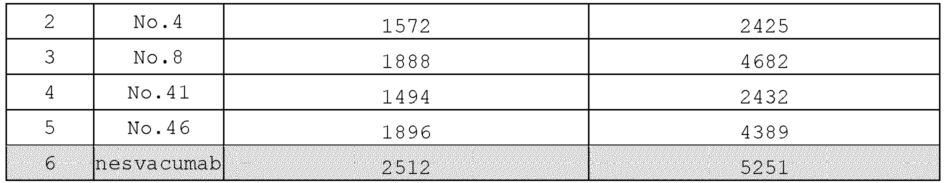

### Example 8. Analysis of Effect of Anti-Ang2 Antibodies on Inhibiting Ang2/Tie2 Signaling (p-Tie2 assay)

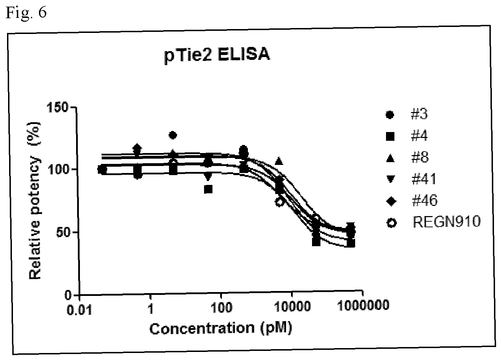

Human Tie2-overexpressing cells (1 × 10⁵) are placed in a 96-well plate and grown overnight at 37 °C in an incubator supplied with carbon dioxide. The cells are grown overnight in a serum-free medium to form a serum deprivation condition. Human Ang2 (5 µg/ml) and various concentrations of anti-Ang2 were allowed to previously react at room temperature for 1 hour, which are then placed in a plate containing the cells, and a reaction was allowed to occur for 20 minutes. In this regard, a well not including the antibody and including only the Ang2 protein is included in the plate and used as a reference value for the analysis of a signaling inhibitory effect. The cells were lysed with lysis buffer and then quantified. To measure a phosphorylation reaction, Human Phospho-Tie2 Duoset IC ELISA (R&D Systems, DYC2720-5) available from R&D Systems was used. 4 ug/ml of human Tie2 capture protein was added to each well of a 96-well immunoplate (Nunc, US) and then allowed to stand overnight at 4 °C for adsorption. The next day, 200 µl of a diluent was added to each well and allowed to stand at room temperature for 2 hours to perform blocking. 50 µg of the cell lysate was added and binding was allowed to occur at room temperature for 2 hours. After the reaction was completed, anti-phosphotyrosine antibodies were diluted to 2700:1 and binding was allowed to occur at room temperature for 2 hours. For the reaction-completed plate, color development was performed using a TMB substrate reagent. The color development reaction was stopped by adding 50 µl of 2N H₂SO₄, and a Sunrise microplate reader (TECAN, CH) was used to measure specific absorbance OD₄₅₀ (FIG. 6). As illustrated in FIG. 6, it can be confirmed that, as the concentration of the antibody is increased, phosphorylation is reduced. The degree of phosphorylation was quantified by obtaining IC₅₀ values, and the results thereof are shown in Table 8.

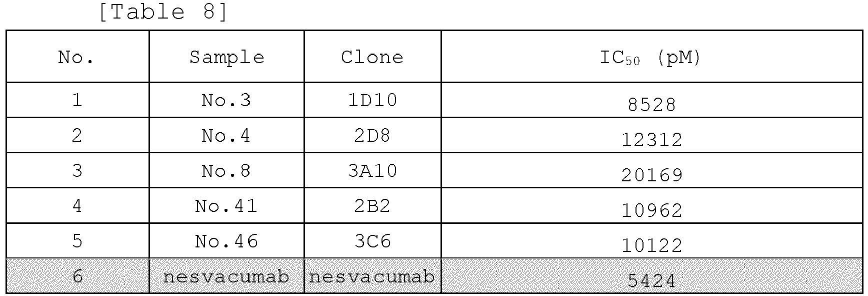

### Example 9: Construction and Selection of Variants for Affinity Enhancement

Antibody optimization was performed to enhance the affinity of anti-Ang2 antibody clone No. 8. By using a soft-randomization method for conserving and randomizing 70% of the original DNA sequence of No. 8, primers having random mutations introduced into light chain CDR3 and heavy chain CDR3 of No. 8 were prepared. Through PCR using the primers, DNA fragments encoding the mutation-introduced light chain variable region and heavy chain variable region were obtained. The DNA fragments were respectively substituted with the light chain variable region of No. 8 scFv phagemid and the heavy chain variable region of No. 8 scFv phagemid, thereby completing the construction of a scFv phage DNA library of light chain CDR3 and heavy chain CDR3 variants.

The scFv phage DNA library was purified with phenolchloroform, and then transformed into the *E. coli* strain XL-1 Blue using electroporation. After confirming that diversity was obtained through transformation efficiency analysis and DNA sequencing, 500 ml of the strain was cultured to induce phage expression, and PEG-precipitation was used to construct scFv phage libraries of light chain and heavy chain CDR3 variants.

Biopanning was performed using the method according to Example 1 using each mutant scFv phage library. Thereafter, in a screening process, the dissociation rate constant kdis was measured as a quantitative evaluation index for the ability to maintain binding. The amino acid sequences of three selected and optimized clones (Tables 9 and 10) and the dissociation rate constant measurement results (Table 11) are shown.

**[Table 9]**

| Sequence number | | Amino acid sequence | SEQ ID NO: |
|---|---|---|---|
| No.04 | Heavy-chain CDR3 | AKTLAGYSGPMGGMDV | 51 |
| No.010 | Heavy-chain CDR3 | AKILVGYSGPMGGMDV | 52 |
| No.012 | Heavy-chain CDR3 | AKSLASYSGPMGGMDV | 53 |

**[Table 10]**

| Antibody name | | Sequence | SEQ ID NO: |
|---|---|---|---|
| No.04 | Heavy chain variable region | CAGGTGCAGCTGGTAGAGTCTGGGGGAGG CCTGGTCAAGCCTGGGGGGTCCCTGAGAC TCTCCTGTGCAGCCTCTGGATTCACCTTC AGTAGCTATAGCATGAACTGGGTCCGCCA GGCTCCAGGGAAGGGGCTGGAGTGGGTCT CATCCATTAGTGCTAGTGATGGTGCCACA TACTACGCAGACTCCGTGAGGGGCCGGTT CACCATCTCCAGAGACAATTCCAGGAGCA CACTGTATCTGCAAATGAACAGTCTGAGA GCCGAGGACACGGCCGTGTATTACTGTGC GAAAACTCTCGCGGGATATAGTGGCCCAA TGGGTGGCATGGACGTCTGGGGCCAAGGG ACCACGGTCACCGTCTCCTCA | 54 |
| | | QVQLVESGGGLVKPGGSLRLSCAASGFTF SSYSMNWVRQAPGKGLEWVSSISASDGAT YYADSVRGRFTISRDNSRSTLYLQMNSLR AEDTAVYYCAKTLAGYSGPMGGMDVWGQG TTVTVSS | 55 |
| | Light chain variable region | GACATCCAGATGACCCAGTCTCCATCCTC ACTGTCTGCATCTGTAGGAGACAGAGTCA CCATCACTTGTCGGGCGAGTCGGGACATT AGCAACTATTTAGCCTGGTATCAGCAGAA ACCAGGGAAAGCCCCTAAGTCCCTGATCT ATGGAGCATCCAATTTACAAAGTGGGGTC TCATCACAGTTCAGCGGCAGTGGATCCGG GACAGATTTCACCCTCACCATCAACAGCC TGCAGCCTGAAGATTCTGCAACTTATTAC TGTCAACAGTACTATAGTTACCCGCTCAC | 41 |
| | | TTTTGGCGGAGGGACCAAGGTGGATATCA AACGT | |
| | | DIQMTQSPSSLSASVGDRVTITCRASRDI SNYLAWYQQKPGKAPKSLIYGASNLQSGV SSQFSGSGSGTDFTLTINSLQPEDSATYY CQQYYSYPLTFGGGTKVDIKR | 42 |
| No.010 | Heavy chain variable region | CAGGTGCAGCTGGTAGAGTCTGGGGGAGG CCTGGTCAAGCCTGGGGGGTCCCTGAGAC TCTCCTGTGCAGCCTCTGGATTCACCTTC AGTAGCTATAGCATGAACTGGGTCCGCCA GGCTCCAGGGAAGGGGCTGGAGTGGGTCT CATCCATTAGTGCTAGTGATGGTGCCACA TACTACGCAGACTCCGTGAGGGGCCGGTT CACCATCTCCAGAGACAATTCCAGGAGCA CACTGTATCTGCAAATGAACAGTCTGAGA GCCGAGGACACGGCCGTGTATTACTGTGC GAAAATCCTCGTAGGATACAGTGGCCCAA TGGGCGGAATGGACGTCTGGGGCCAAGGG ACCACGGTCACCGTCTCCTCA | 56 |
| | | QVQLVESGGGLVKPGGSLRLSCAASGFTF SSYSMNWVRQAPGKGLEWVSSISASDGAT YYADSVRGRFTISRDNSRSTLYLQMNSLR AEDTAVYYCAKILVGYSGPMGGMDVWGQG TTVTVSS | 57 |
| | Light chain variable region | GACATCCAGATGACCCAGTCTCCATCCTC ACTGTCTGCATCTGTAGGAGACAGAGTCA CCATCACTTGTCGGGCGAGTCGGGACATT AGCAACTATTTAGCCTGGTATCAGCAGAA ACCAGGGAAAGCCCCTAAGTCCCTGATCT ATGGAGCATCCAATTTACAAAGTGGGGTC TCATCACAGTTCAGCGGCAGTGGATCCGG GACAGATTTCACCCTCACCATCAACAGCC TGCAGCCTGAAGATTCTGCAACTTATTAC TGTCAACAGTACTATAGTTACCCGCTCAC TTTTGGCGGAGGGACCAAGGTGGATATCA AACGT | 41 |
| | | DIQMTQSPSSLSASVGDRVTITCRASRDI SNYLAWYQQKPGKAPKSLIYGASNLQSGV SSQFSGSGSGTDFTLTINSLQPEDSATYY CQQYYSYPLTFGGGTKVDIKR | 42 |
| No.012 | Heavy chain variable region | CAGGTGCAGCTGGTAGAGTCTGGGGGAGG CCTGGTCAAGCCTGGGGGGTCCCTGAGAC TCTCCTGTGCAGCCTCTGGATTCACCTTC AGTAGCTATAGCATGAACTGGGTCCGCCA GGCTCCAGGGAAGGGGCTGGAGTGGGTCT CATCCATTAGTGCTAGTGATGGTGCCACA TACTACGCAGACTCCGTGAGGGGCCGGTT CACCATCTCCAGAGACAATTCCAGGAGCA CACTGTATCTGCAAATGAACAGTCTGAGA GCCGAGGACACGGCCGTGTATTACTGTGC GAAAAGCCTTGCCAGCTATAGTGGCCCAA | 58 |
| | | TGGGCGGCATGGACGTCTGGGGCCAAGGG ACCACGGTCACCGTCTCCTCA | |
| | | QVQLVESGGGLVKPGGSLRLSCAASGFTF SSYSMNWVRQAPGKGLEWVSSISASDGAT YYADSVRGRFTISRDNSRSTLYLQMNSLR AEDTAVYYCAKSLASYSGPMGGMDVWGQG TTVTVSS | 59 |
| | Light chain variable region | GACATCCAGATGACCCAGTCTCCATCCTC ACTGTCTGCATCTGTAGGAGACAGAGTCA CCATCACTTGTCGGGCGAGTCGGGACATT AGCAACTATTTAGCCTGGTATCAGCAGAA ACCAGGGAAAGCCCCTAAGTCCCTGATCT ATGGAGCATCCAATTTACAAAGTGGGGTC TCATCACAGTTCAGCGGCAGTGGATCCGG GACAGATTTCACCCTCACCATCAACAGCC TGCAGCCTGAAGATTCTGCAACTTATTAC TGTCAACAGTACTATAGTTACCCGCTCAC TTTTGGCGGAGGGACCAAGGTGGATATCA AACGT | 41 |
| | | DIQMTQSPSSLSASVGDRVTITCRASRDI SNYLAWYQQKPGKAPKSLIYGASNLQSGV SSQFSGSGSGTDFTLTINSLQPEDSATYY CQQYYSYPLTFGGGTKVDIKR | 42 |
| No.04-1 | Heavy chain variable region | CAGGTGCAGCTGGTAGAGTCTGGGGGAGG CCTGTTCAAGCCTGGGGGGTCCCTGAGAC TCTCCTGTGCAGCCTCTGGATTCACCTTC AGTAGCTATAGCATGAACTGGGTCCGCCA GGCTCCAGGGAAGGGGCTGGAGTGGGTCT CATCCATTAGTGCTAGTGATGGTGCCACA TACTACGCAGACTCCGTGAGGGGCCGGTT CACCATCTCCAGAGACAATTCCAGGAGCA CACTGTATCTGCAAATGAACAGTCTGAGA GCCGAGGACACGGCCGTGTATTACTGTGC GAAAACTCTCGCGGGATATAGTGGCCCAA TGGGTGGCATGGACGTCTGGGGCCAAGGG ACCACGGTCACCGTCTCCTCA | 60 |
| | | QVQLVESGGGLSKPGGSLRLSCAASGFTF SSYSMNWVRQAPGKGLEWVSSISASDGAT YYADSVRGRFTISRDNSRSTLYLQMNSLR AEDTAVYYCAKTLAGYSGPMGGMDVWGQG TTVTVSS | 61 |
| | Light chain variable region | GACATCCAGATGACCCAGTCTCCATCCTC ACTGTCTGCATCTGTAGGAGACAGAGTCA CCATCACTTGTCGGGCGAGTCGGGACATT AGCAACTATTTAGCCTGGTATCAGCAGAA ACCAGGGAAAGCCCCTAAGTCCCTGATCT ATGGAGCATCCAATTTACAAAGTGGGGTC TCATCACAGTTCAGCGGCAGTGGATCCGG GACAGATTTCACCCTCACCATCAACAGCC TGCAGCCTGAAGATTCTGCAACTTATTAC TGTCAACAGTACTATAGTTACCCGCTCAC | 41 |
| | | TTTTGGCGGAGGGACCAAGGTGGATATCA AACGT | |
| | | DIQMTQSPSSLSASVGDRVTITCRASRDI SNYLAWYQQKPGKAPKSLIYGASNLQSGV SSQFSGSGSGTDFTLTINSLQPEDSATYY CQQYYSYPLTFGGGTKVDIKR | 42 |

**[Table 11 ]**

| Clone | Kdis (1/s) |
|---|---|
| No.04 | 9.01E-05 |
| No.010 | 3.92E-05 |
| No.012 | 1.70E-05 |

### Example 10: ScFv Production of Optimized Anti-Ang2 Antibody

The optimized anti-Ang2 antibody (No.04) was cloned into pET-22b vector (Novagen) for expression in *E. coli.* Colonies generated by transforming the vector into BL21 (De3) were selected, 100 ug/ml of ampicillin was added to LB (Lysogeny broth) medium, and 1% of *E. coli* pre-cultured at 37 °C and 200 rpm was inoculated into the LB medium containing 100 ug/ml ampicillin as an antibiotic. The *E. coli* was incubated at 37 °C and 200 rpm, the temperature of an incubator was lowered to 20 °C, and 0.5 mM IPTG was added, followed by incubation for 16 hours.

The incubated *E. coli* was collected by centrifugation at 8000 rpm for 10 minutes. After removing the medium, resuspension was performed using 10 ml (per the weight (g) of cells) of a lysis buffer containing 50 mM Tris-HCl pH 7.4, 150 mM NaCl, and 1 mM Phenylmethylsulfonyl fluoride (PMSF). The cells were disrupted using an ultrasonic processor under conditions of power: 20 W, rest: 3 sec, work: 3 sec, and time: 5 min. The disrupted cells were centrifuged at 11000 rpm for 1 hour to separate the supernatant and the precipitate.

ScFv was expressed in an insoluble form, and pellet washing was performed for refolding. After homogenizing with a homogenizer using 50 mM Tris-HCl pH 7.4, 150 mM NaCl buffer, the pellet was washed twice by centrifugation at 11000 rpm for 1 hour. *E*. *coli*-derived substances remaining in the pellet were removed using 50 mM Tris-HCl pH 7.4, 150 mM NaCl, 2 M Urea, 0.5% Triton X-100 buffer, and washing was repeated three times with 50 mM Tris-HCl pH 7.4, 150 mM NaCl buffer. The inclusion body was re-suspended using 50 mM Tris-HCl pH 7.4, 150 mM NaCl, 8 M Urea, 10 mM DTT buffer, and then a reaction was allowed to occur for about 30 minutes to produce scFv in an unfolded form, followed by centrifugation at 11000 rpm for 1 hour to separate the precipitate.

The scFv antibody was subjected to step dialysis to remove urea, thereby inducing refolding. The dialysis was performed using a dialysis buffer basically containing 50 mM Tris-HCl pH 7.4 and 150 mM NaCl by reducing the concentration of urea by 1/2. Refolding was performed by suppressing aggregation by adding 0.1 M L-arginine to the 4-2-1 M urea concentration fractions where the proper protein folding is mostly formed. The refolded scFv antibody was separated and purified using HisTrap and Capo L columns.

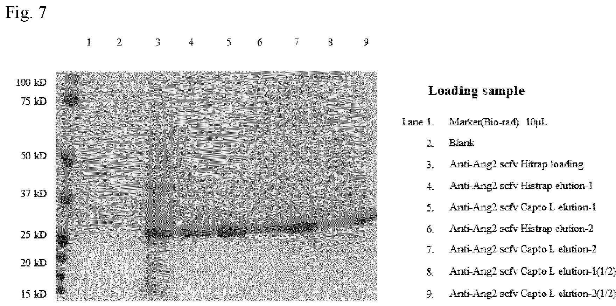

Separation and purification was performed in the following order. Purification was performed using AKTA Prime (GE Healthcare) System, and 5 ml of a HisTrap packing column was used. 10-column volumes of 50 mM Sodium Phosphate, 400 mM NaCl, 10 mM Imidazole pH 7.4 buffer was flowed into the HisTrap column for equilibrium, and the scFv antibody sample was allowed to flow into the HisTrap column at a flow rate of 5 ml/min to allow the antibody sample to bind to a resin inside the column. In order to remove non-specific binding substances present in the resin, about 10 column volumes of 50 mM sodium Phosphate, 400 mM NaCl, 10 mM Imidazole pH 7.4 buffer were allowed to flow, and then 50 mM Sodium Phosphate, 400 mM NaCl, 300 mM Imidazole pH 7.4 buffer was allowed to flow with concentration gradients so that the scFv antibody was eluted. The eluted antibody sample was subjected to the following purification using 5 ml of Capto L column. The Capto L column was equilibrated with phosphate-buffered saline (PBS) pH 7.4 buffer, and then the sample was allowed to flow into the Capto L column at a flow rate of 5 ml/min to remove the non-specific binding substances. 10 column volumes of 0.1 M Citric Acid, 0.2 M Na2HPO4 pH 2.6 buffer were allowed to flow so that the scFv antibody was eluted. FIG. 7 illustrates SDS-PAGE results of the protein, which was finally obtained after purification. As a result, the obtained scFv antibody exhibited high purity.

### Example 11: Analysis of Binding Specificity of Optimized Anti-Ang2 ScFv Antibody

For binding specificity analysis, binding constants were measured using an ELISA method and an Octet system (Pall ForteBio LLC, US).

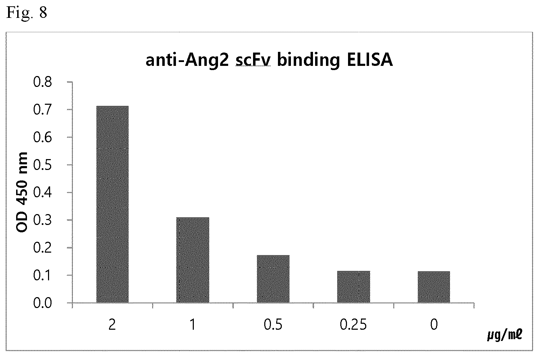

1 ug/ml (100 µl per well) of a His-tagged human Ang2 protein (R&D systems, 623-AN/CF) solution was added to a 96-well immunoplate (Nunc, US) and allowed to stand overnight at 4 °C for adsorption. The next day, the solution was washed three times with PBS containing 0.05% Tween-20 (hereinafter, referred to as PBST), and 200 µl of a 2% BSA/PBST solution was added to each well and then allowed to stand at room temperature for 2 hours to perform blocking. After washing three times with PBST, 100 µl of each test antibody solution was added to each well according to concentration to cause binding at room temperature for 1 hour, and then washing was performed three times with PBST, 100 µl of HRP-conjugated anti-His tag monoclonal antibody (MAB050H, R&D Systems, US) diluted to 1:1000 was added and allowed to react at room temperature for 1 hour, thereby inducing binding, and after washing three times with PBST, color development was performed using 100 µl of a TMB substrate reagent. The color development reaction was stopped by adding 50 µl of 2N H₂SO₄, and a Sunrise microplate reader (TECAN, CH) was used to measure specific absorbance OD₄₅₀ (FIG. 8). As illustrated in FIG. 8, the purified antibody binds to human Ang2.

The binding affinity of the purified scFv antibody to human Ang2 was measured using an Octet system (ForteBio Inc., US) . To this end, the anti-Ang2 antibody was immobilized on a biosensor, and binding kinetics of human Ang2 according to concentration were measured to calculate binding rate constant (kₐ), dissociation rate constant (k_{dis}), and binding constant (K_{D}) (Table 12).

**[Table 12]**

| Sample | kₐ (1/Ms) | k_{d}(1/s) | K_{D} (M) |
|---|---|---|---|
| No.04 scFv | 9.4E+04 | 2.54E-05 | 2.8E-10 |

### Example 12: In-Vivo Efficacy Analysis of Selected Anti-Ang2 ScFv Antibodies (CNV Mouse Model)

To confirm whether the anti-Ang2 scFv antibody has inhibitory efficacy against angiogenesis, a drug efficacy test was performed in a laser-induced choroidal neovascularization mouse model. The efficacy was tested using aflibercept as a control, which is a commercially available drug. Mice were generally anesthetized with ketamine, and then anesthetic eye drops were applied to the eyeballs for additional local anesthesia, and a mydriatic was applied to the eyes to induce mydriasis. Each mouse was placed on a stage and Micron-IV was used to induce a laser burn under CNV induction conditions (wavelength: 532 nm, diameter: 50 µm, duration: 80 mS, and power level: 200 mW), thereby destroying the Bruch's membrane. In the laser burn induction process, lesions where bubbling was not observed were classified as unsuccessful laser burns and excluded from result analysis and statistical processing on the basis of exclusion criteria obtained by modifying criteria proposed by Gong Y. et al.

To confirm the angiogenesis inhibitory effect by CNV induction and drugs, mice were generally anesthetized with Ketamine^{®} on day 10 after CNV induction, and then a fluorescent contrast agent was intraperitoneally injected into each mouse. Anesthetic eye drops were applied to the eyeballs for additional local anesthesia, and a mydriatic was applied to the eyes to induce mydriasis. Each mouse was placed on a stage, fundus image alignment was performed using an imaging camera of Micron-IV, and then lubricating gel was applied to the corresponding eye and OCT lens was brought into contact with the cornea of each mouse. After FFA/OCT imaging, 1 drop of antibiotic eye drop was applied to the eyes of the mice. Analysis for FFA and OCT images was performed using the "Image-J" program. In this regard, CNV lesions corresponding to the exclusion criteria proposed by Gong Y. et al. were excluded from the final results and statistical analysis.

To confirm the recovery effect of the optic nerve, an electroretinogram (ERG) test was performed. Mice were placed in a dark room 12 hours before ERG evaluation to induce dark adaptation. On the day of evaluation (day 11 after CNV induction), the mice were generally anesthetized with Rompun^{®} and Ketamine^{®}, and then Alcaine^{®} was applied to the eyes for additional local anesthesia, and a mydriatic was applied to the eyes to induce mydriasis. Each mouse was placed on an ERG stage and probes of ERG were brought into contact with the tail, head, and cornea, respectively. ERG was measured as a change in retinal potential for a single flash stimulus (0.9 log cds/m2 (10 responses/intensity)). When the ERG evaluation was completed, 1 drop of Tobrex was applied to the eyes of the mice. ERG analysis was performed using a LabScribeERG (iWorx Data Acquisition Software) program. In this regard, eyes corresponding to the exclusion criteria proposed by Gong Y. et al. were excluded from the final results and statistical analysis.

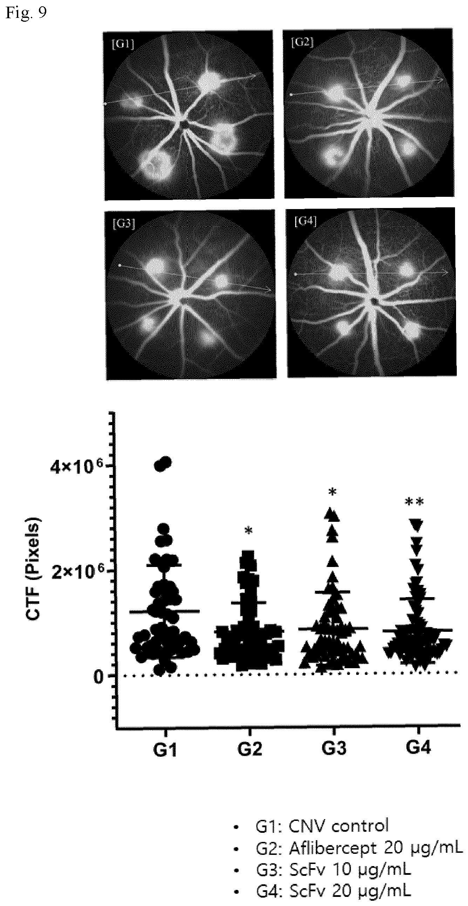

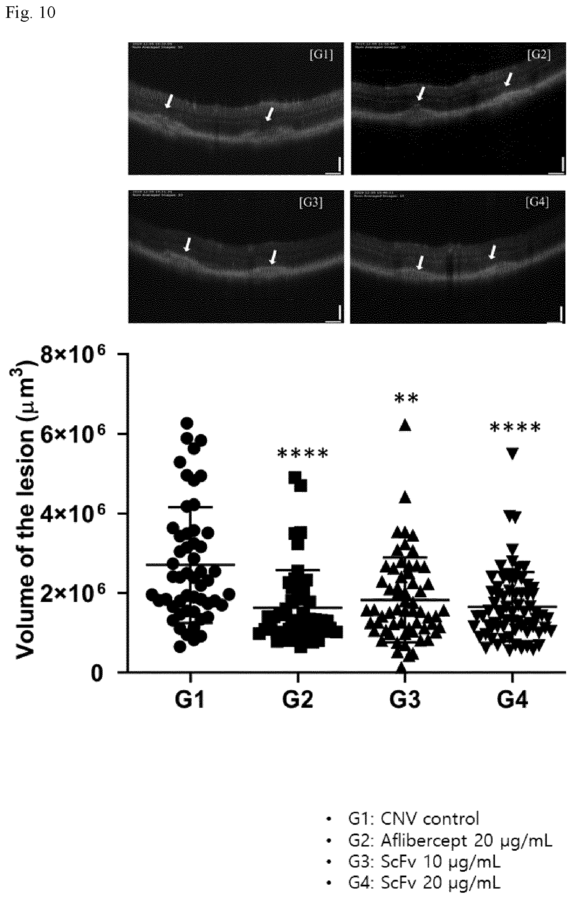

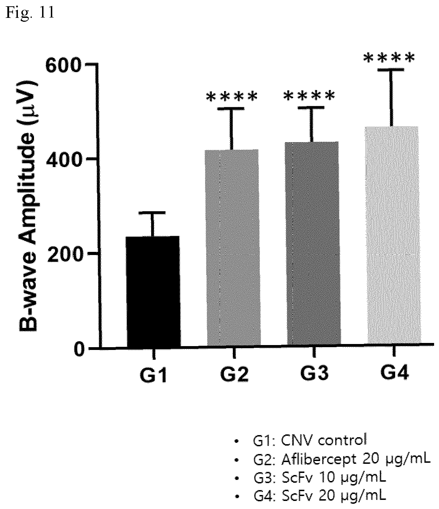

The results confirming the efficacy of the anti-Ang2 scFv antibody on reducing angiogenesis in a CNV mouse model are shown in FIG. 9. A statistically significant decrease in the size of CNV lesion observed on FFA was observed in an experimental group administered 10 µg/µL of scFv (P<0.01), showing a more potent effect compared to an experimental group administered aflibercept. From FIG. 10, it was confirmed that, even when compared to the CNV control by OCT measurement, all experimental groups administered scFv exhibited a statistically significant decrease in the volume of the lesion in a concentration-dependent manner (P<0.01, P<0.0001, P<0.05, respectively). In addition, as illustrated in FIG. 11, when the retinal potential was compared with that of the CNV control by scotopic-ERG, a statistically significant increase in B-wave amplitude was observed (P<0.0001, respectively).

### Example 13: Analysis of Antitumor Efficacy of Selected Anti-Ang2 Antibody (TNBC model)

To confirm whether the anti-Ang2 antibody has inhibitory efficacy against angiogenesis, an antitumor efficacy test was conducted in a human triple-negative breast cancer (TNBC) model (MDA-MB-231). In order to determine the antitumor activity of the anti-Ang2 antibody, the anticancer efficacies of isotype control, nesvacumab, and the anti-Ang2 antibody by injection into the tail vein in NSG mice having the human-derived breast cancer cell line MDA-MB-231 transplanted at the left flank thereof were evaluated. Champions Oncology, Inc. (US) was requested to perform the experiment.

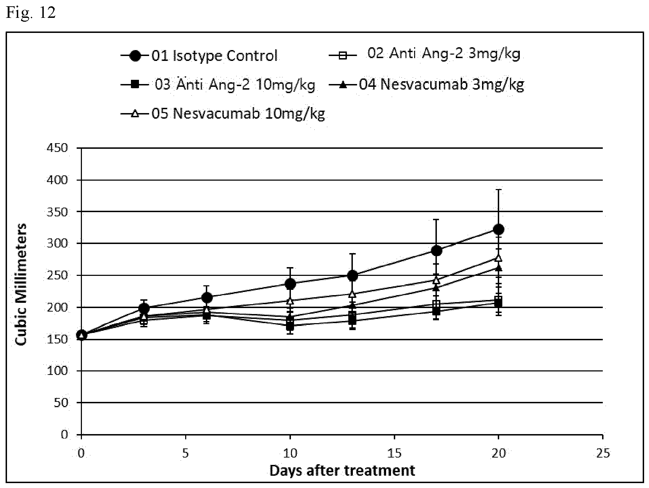

The human breast cancer cell line MDA-MB-231 (breast cancer cell line) was thawed and cultured in a CO₂ incubator (Forma, USA) at a temperature of 37 °C and a CO₂ concentration of 5%. On the last day of culture, all cancer cells were collected and counted, and the cell concentration was adjusted to 1×10⁸ cells/ml using serum-free media. The adjusted cell culture solution was injected into the left flank of each mouse at 0.1 ml (1×10⁷ cells/mouse). The mice were grouped into 8 mice per treated group in a zigzag manner until the volume of tumors reached 100-150 mm³. Immediately after grouping, drug administration began. 10 mg/kg of isotype control as a negative control, 3 mg/kg or 10 mg/kg of an anti-Ang2 antibody, and 3 mg/kg or 10 mg/kg of Nesvacumab were administered via intravenous injection twice a week for 3 weeks. After drug administration, the volume of tumors was measured twice a week for 20 days. During the measurement process, the mice did not exhibit clinical toxic responses. The group treated with 10 mg/kg of anti-Ang2 antibody exhibited an effect of inhibiting tumor growth by about 70%, which is about twice that of the group treated with 10 mg/kg of Nesvacumab. The tumor volume was calculated using the following equation: Tumor volume (TV) = width² × length × 0.52. The weight of each individual showed a uniform increase of about 5% on average, from which it was confirmed that there was no particular toxic reaction (FIG. 12).

**[Table 13] Effect of inhibiting tumor growth by administration of anti-Ang2 antibody (mm³)**

| **Tumor Volume - Mean** | | | | | | | |
|---|---|---|---|---|---|---|---|
| **Treatment** | 0 | 3 | 6 | 10 | 13 | 17 | 20 |
| 01 Isotype Control | 157 | 199 | 216 | 237 | 250 | 290 | 323 |
| 02 anti-Ang2 3mg/kg | 157 | 179 | 187 | 179 | 188 | 205 | 212 |
| 03 anti-Ang2 10mg/kg | 157 | 184 | 188 | 171 | 178 | 193 | 207 |
| 04 Nesvacumab 3mg/kg | 157 | 186 | 192 | 185 | 203 | 231 | 262 |
| 05 Nesvacumab 10mg/kg | 157 | 186 | 197 | 210 | 221 | 243 | 278 |
| | | | | | | | |

| **Tumor Volume - SEM** | | | | | | | |
|---|---|---|---|---|---|---|---|
| **Treatment** | 0 | 3 | 6 | 10 | 13 | 17 | 20 |
| 01 Isotype Control | 7.254 | 12.682 | 18.216 | 25.044 | 34.67 | 47.517 | 61.862 |
| 02 anti-Ang23mg/kg | 6.777 | 9.424 | 12.741 | 14.444 | 20.313 | 23.01 | 25.324 |
| 03 anti-Ang2 10mg/kg | 6.625 | 10.606 | 10.416 | 13.104 | 12.733 | 12.434 | 15.184 |
| 04 Nesvacumab 3mg/kg | 6.155 | 7.339 | 8.681 | 13.944 | 17.324 | 21.042 | 29.726 |
| 05 Nesvacumab 10mg/kg | 6.009 | 12.15 | 12.827 | 18.151 | 19.917 | 25.11 | 31.335 |
| | | | | | | | |

| **Tumor Growth Inhibition (%)** | | | | | | | |
|---|---|---|---|---|---|---|---|
| **Treatment** | 0 | 3 | 6 | 10 | 13 | 17 | 20 |
| 01 Isotype Control | 0 | 0 | 0 | 0 | 0 | 0 | 0 |
| 02 anti-Ang2 3mg/kg | 0 | 47.62 | 49.15 | 72.5 | 66.67 | 63.91 | 66.87 |
| 03 anti-Ang2 10mg/kg | 0 | 35.71 | 47.46 | 82.5 | 77.42 | 72.93 | 69.88 |
| 04 Nesvacumab 3mg/kg | 0 | 30.95 | 40.68 | 65 | 50.54 | 44.36 | 36.75 |
| 05 Nesvacumab 10mg/kg | 0 | 30.95 | 32.2 | 33.75 | 31.18 | 35.34 | 27.11 |

**[Table 14] Changes in body weight (g) of mice according to date**

| **Body Weight - Mean** | | | | | | | |
|---|---|---|---|---|---|---|---|
| **Treatment** | 0 | 3 | 6 | 10 | 13 | 17 | 20 |
| 01 Isotype Control | 23.281 | 23.707 | 23.736 | 23.825 | 24.259 | 24.478 | 24.489 |
| 02 anti-Ang2 3mg/kg | 23.356 | 23.701 | 24.449 | 24.444 | 25.19 | 25.23 | 25.201 |
| 03 anti-Ang2 10mg/kg | 24.046 | 24.482 | 24.899 | 24.423 | 25.161 | 25.355 | 25.433 |
| 04 Nesvacumab 3mg/kg | 23.253 | 23.813 | 24.198 | 23.991 | 24.768 | 25.066 | 25.186 |
| 05 Nesvacumab 10mg/kg | 22.648 | 23.531 | 23.96 | 23.53 | 24.159 | 24.674 | 24.346 |
| | | | | | | | |

| **Body Weight - SEM** | | | | | | | |
|---|---|---|---|---|---|---|---|
| **Treatment** | 0 | 3 | 6 | 10 | 13 | 17 | 20 |
| 01 Isotype Control | 1.38 | 1.042 | 1.02 | 1.129 | 0.97 | 1.066 | 1.117 |
| 02 anti-Ang2 3mg/kg | 0.473 | 0.411 | 0.474 | 0.539 | 0.555 | 0.582 | 0.556 |
| 03 anti-Ang2 10mg/kg | 0.751 | 0.705 | 0.769 | 0.829 | 0.85 | 0.889 | 0.86 |
| 04 Nesvacumab 3mg/kg | 0.772 | 0.658 | 0.771 | 0.807 | 0.788 | 0.659 | 0.918 |
| 05 Nesvacumab 10mg/kg | 0.346 | 0.308 | 0.238 | 0.318 | 0.312 | 0.359 | 0.502 |

### [Industrial Applicability]

An anti-Ang2 antibody or antigen-binding fragment thereof according to the present disclosure exhibits a desired ability to bind to Ang2, and can be effectively used in inhibitors for cancer/tumor or angiogenesis and the prevention or treatment of diseases related to angiopoietin-2 activation and/or overproduction. According to the present disclosure, by developing therapeutic agents having target points different from those of existing Ang2-targeting therapeutic agents, a combination treatment with an existing therapeutic agent and a single treatment can be provided for the treatment of tumors.

While specific embodiments of the present disclosure have been described in detail, it will be obvious to those of ordinary skill in the art that these detailed descriptions are merely exemplary embodiments and are not intended to limit the scope of the present disclosure. Therefore, the substantial scope of the present disclosure should be defined by the appended claims and equivalents thereto.

## Claims

1. An antibody binding to angiopoietin-2 (Ang2) or an antigen-binding fragment thereof, comprising:
a heavy chain variable region comprising:
a heavy chain CDR1 selected from the group consisting of SEQ ID NOS: 1, 7, 13, 19, and 25;
a heavy chain CDR2 selected from the group consisting of SEQ ID NOS: 2, 8, 14, 20, and 26; and
a heavy chain CDR3 selected from the group consisting of SEQ ID NOS: 3, 9, 15, 21, 27, 51, 52, and 53; and
a light chain variable region comprising:
a light chain CDR1 selected from the group consisting of SEQ ID NOS: 4, 10, 16, 22, and 28;
a light chain CDR2 selected from the group consisting of SEQ ID NOS: 5, 11, 17, 23, and 29; and
a light chain CDR3 selected from the group consisting of SEQ ID NOS: 6, 12, 18, 24, and 30.

2. The antibody or antigen-binding fragment thereof according to claim 1, wherein the antibody or the antigen-binding fragment thereof comprises:
a heavy chain variable region comprising a heavy chain CDR1 of SEQ ID NO: 1, a heavy chain CDR2 of SEQ ID NO: 2, and a heavy chain CDR3 of SEQ ID NO: 3, and a light chain variable region comprising a light chain CDR1 of SEQ ID NO: 4, a light chain CDR2 of SEQ ID NO: 5, and a light chain CDR3 of SEQ ID NO: 6;
a heavy chain variable region comprising a heavy chain CDR1 of SEQ ID NO: 7, a heavy chain CDR2 of SEQ ID NO: 8, and a heavy chain CDR3 of SEQ ID NO: 9, and a light chain variable region comprising a light chain CDR1 of SEQ ID NO: 10, a light chain CDR2 of SEQ ID NO: 11, and a light chain CDR3 of SEQ ID NO: 12;
a heavy chain variable region comprising a heavy chain CDR1 of SEQ ID NO: 13, a heavy chain CDR2 of SEQ ID NO: 14, and a heavy chain CDR3 of SEQ ID NO: 15, and a light chain variable region comprising a light chain CDR1 of SEQ ID NO: 16, a light chain CDR2 of SEQ ID NO: 17, and a light chain CDR3 of SEQ ID NO: 18;
a heavy chain variable region comprising a heavy chain CDR1 of SEQ ID NO: 19, a heavy chain CDR2 of SEQ ID NO: 20, and a heavy chain CDR3 of SEQ ID NO: 21, and a light chain variable region comprising a light chain CDR1 of SEQ ID NO: 22, a light chain CDR2 of SEQ ID NO: 23, and a light chain CDR3 of SEQ ID NO: 24;
a heavy chain variable region comprising a heavy chain CDR1 of SEQ ID NO: 25, a heavy chain CDR2 of SEQ ID NO: 26, and a heavy chain CDR3 of SEQ ID NO: 27, and a light chain variable region comprising a light chain CDR1 of SEQ ID NO: 28, a light chain CDR2 of SEQ ID NO: 29, and a light chain CDR3 of SEQ ID NO: 30;
a heavy chain variable region comprising a heavy chain CDR1 of SEQ ID NO: 13, a heavy chain CDR2 of SEQ ID NO: 14, and a heavy chain CDR3 of SEQ ID NO: 51, and a light chain variable region comprising a light chain CDR1 of SEQ ID NO: 16, a light chain CDR2 of SEQ ID NO: 17, and a light chain CDR3 of SEQ ID NO: 18;
a heavy chain variable region comprising a heavy chain CDR1 of SEQ ID NO: 13, a heavy chain CDR2 of SEQ ID NO: 14, and a heavy chain CDR3 of SEQ ID NO: 52, and a light chain variable region comprising a light chain CDR1 of SEQ ID NO: 16, a light chain CDR2 of SEQ ID NO: 17, and a light chain CDR3 of SEQ ID NO: 18; or
a heavy chain variable region comprising a heavy chain CDR1 of SEQ ID NO: 13, a heavy chain CDR2 of SEQ ID NO: 14, and a heavy chain CDR3 of SEQ ID NO: 53, and a light chain variable region comprising a light chain CDR1 of SEQ ID NO: 16, a light chain CDR2 of SEQ ID NO: 17, and a light chain CDR3 of SEQ ID NO: 18.

3. The antibody or antigen-binding fragment thereof according to claim 1, wherein the antibody or the antigen-binding fragment thereof comprises a heavy chain variable region selected from the group consisting of SEQ ID NOS: 32, 36, 40, 44, 48, 55, 57, 59, and 61.

4. The antibody or antigen-binding fragment thereof according to claim 1, wherein the antibody or the antigen-binding fragment thereof comprises a light chain variable region selected from the group consisting of SEQ ID NOS: 34, 38, 42, 46, and 50.

5. A nucleic acid encoding the antibody or antigen-binding fragment thereof of any one of claims 1 to 4.

6. The nucleic acid according to claim 5, wherein the nucleic acid is selected from the group consisting of SEQ ID NOS: 31, 35, 39, 43, 47, 54, 56, 58, and 60 encoding a heavy chain variable region.

7. The nucleic acid according to claim 5, wherein the nucleic acid is selected from the group consisting of SEQ ID NOS: 33, 37, 41, 45, and 49 encoding a light chain variable region.

8. An expression vector comprising the nucleic acid of claim 5.

9. A cell transformed with the expression vector of claim 8.

10. A method of producing an antibody binding to Ang2 or an antigen-binding fragment thereof, the method comprising the following processes:
(a) culturing the cells of claim 9; and
(b) recovering the antibody or the antigen-binding fragment thereof from the cultured cells.

11. A composition comprising the antibody or an antigen-binding fragment thereof according to any one of claims 1 to 4 as an active ingredient for preventing or treating a disease related with angiopoietin-2 activation and/or overproduction.

12. A composition comprising the antibody or an antigen-binding fragment thereof according to any one of claims 1 to 4 as an active ingredient for inhibiting an angiogenesis.

13. A composition comprising the antibody or an antigen-binding fragment thereof according to any one of claims 1 to 4 as an active ingredient for diagnosing a disease related with angiopoietin-2 activation and/or overproduction.

14. A composition comprising the antibody or an antigen-binding fragment thereof according to any one of claims 1 to 4 as an active ingredient for preventing or treating an eye disease.

15. A composition comprising the antibody or an antigen-binding fragment thereof according to any one of claims 1 to 4 as an active ingredient for preventing or treating tumor or cancer.

16. A composition comprising the antibody or an antigen-binding fragment thereof according to any one of claims 1 to 4 for combining with other therapeutic drugs.


<110>    Pharmabcine Inc.

<120>    Anti- Ang2 antibody and Use Thereof

<130>    PP-B2372

<150>    KR 19/021812
<151>    2019-02-25

<160>    61

<170>    PatentIn version 3.5

<210>    1
<211>    8
<212>    PRT
<213>    Artificial Sequence

<220>
<223>    Synthetic Sequence


<400>    1
Gly Phe Ser Phe Asp Asp Tyr Ala
1               5


<210>    2
<211>    8
<212>    PRT
<213>    Artificial Sequence

<220>
<223>    Synthetic Sequence


<400>    2
Ile Lys Asp Asp Gly Ser Gln Thr
1               5


<210>    3
<211>    13
<212>    PRT
<213>    Artificial Sequence

<220>
<223>    Synthetic Sequence


<400>    3
Thr Thr Glu Gly Leu Met Asn Gly Leu His Phe Asp Met
1               5                   10


<210>    4
<211>    9
<212>    PRT
<213>    Artificial Sequence

<220>
<223>    Synthetic Sequence

<400>    4
Ser Ser Asn Ile Gly Ala Gly Tyr Asp
1               5

<210>    5
<211>    3
<212>    PRT
<213>    Artificial Sequence

<220>
<223>    Synthetic Sequence

<400>    5
Gly Asn Asn
1

<210>    6
<211>    10
<212>    PRT
<213>    Artificial Sequence

<220>
<223>    Synthetic Sequence

<400>    6
Gln Ser Tyr Asp Ser Arg Leu Gly Val Val
1               5                   10

<210>    7
<211>    8
<212>    PRT
<213>    Artificial Sequence

<220>
<223>    Synthetic Sequence

<400>    7
Gly Tyr Ser Phe Thr Ser Tyr Trp
1               5

<210>    8
<211>    8
<212>    PRT
<213>    Artificial Sequence

<220>
<223>    Synthetic Sequence

<400>    8
Ile Tyr Pro Gly Asn Ser Asp Thr
1               5

<210>    9

<211>    13
<212>    PRT
<213>    Artificial Sequence

<220>
<223>    Synthetic Sequence


<400>    9
Thr Thr Glu Gly Leu Met Asn Gly Leu His Phe Asp Met
1               5                   10


<210>    10
<211>    11
<212>    PRT
<213>    Artificial Sequence

<220>
<223>    Synthetic Sequence


<400>    10
Gln Ser Leu Leu His Ser Leu Gly Asp Asn Tyr
1               5                   10


<210>    11
<211>    3
<212>    PRT
<213>    Artificial Sequence

<220>
<223>    Synthetic Sequence


<400>    11
Leu Gly Ser
1


<210>    12
<211>    10
<212>    PRT
<213>    Artificial Sequence

<220>
<223>    Synthetic Sequence


<400>    12
Met Gln Ser Leu Gln Thr Pro Pro Tyr Thr
1               5                   10


<210>    13
<211>    8
<212>    PRT
<213>    Artificial Sequence

<220>
<223>    Synthetic Sequence

<400>    13
Gly Phe Thr Phe Ser Ser Tyr Ser
1               5

<210>    14
<211>    8
<212>    PRT
<213>    Artificial Sequence

<220>
<223>    Synthetic Sequence

<400>    14
Ile Ser Ala Ser Asp Gly Ala Thr
1               5

<210>    15
<211>    16
<212>    PRT
<213>    Artificial Sequence

<220>
<223>    Synthetic Sequence

<400>    15
Ala Lys Ile Leu Ala Gly Tyr Ser Gly Pro Met Gly Gly Met Asp Val
1               5                   10                  15

<210>    16
<211>    6
<212>    PRT
<213>    Artificial Sequence

<220>
<223>    Synthetic Sequence

<400>    16
Arg Asp Ile Ser Asn Tyr
1               5

<210>    17
<211>    3
<212>    PRT
<213>    Artificial Sequence

<220>
<223>    Synthetic Sequence

<400>    17
Gly Ala Ser
1

<210>    18
<211>    9
<212>    PRT
<213>    Artificial Sequence

<220>
<223>    Synthetic Sequence


<400>    18
Gln Gln Tyr Tyr Ser Tyr Pro Leu Thr
1               5


<210>    19
<211>    8
<212>    PRT
<213>    Artificial Sequence

<220>
<223>    Synthetic Sequence


<400>    19
Gly Phe Ala Phe Gly Arg Tyr Glu
1               5


<210>    20
<211>    8
<212>    PRT
<213>    Artificial Sequence

<220>
<223>    Synthetic Sequence


<400>    20
Ile Asp Thr Gly Gly Gly Ala Lys
1               5


<210>    21
<211>    13
<212>    PRT
<213>    Artificial Sequence

<220>
<223>    Synthetic Sequence


<400>    21
Thr Thr Glu Gly Leu Met Asn Gly Leu His Phe Asp Met
1               5                   10


<210>    22
<211>    6
<212>    PRT
<213>    Artificial Sequence

<220>

<223>    Synthetic Sequence

<400>    22
Gln Ala Ile Ser Thr Trp
1               5

<210>    23
<211>    3
<212>    PRT
<213>    Artificial Sequence

<220>
<223>    Synthetic Sequence

<400>    23
Thr Ala Ser
1

<210>    24
<211>    9
<212>    PRT
<213>    Artificial Sequence

<220>
<223>    Synthetic Sequence

<400>    24
Gln Gln Leu Asn Ser Tyr Pro Tyr Thr
1               5

<210>    25
<211>    8
<212>    PRT
<213>    Artificial Sequence

<220>
<223>    Synthetic Sequence

<400>    25
Gly Phe Thr Phe Asp Asp Cys Ala
1               5

<210>    26
<211>    8
<212>    PRT
<213>    Artificial Sequence

<220>
<223>    Synthetic Sequence

<400>    26
Ile Ser Gly Asn Ser Lys Asn Val
1               5

<210>    27
<211>    11
<212>    PRT
<213>    Artificial Sequence

<220>
<223>    Synthetic Sequence


<400>    27
Ala Arg Asp Pro Ala Tyr Ser Gln Phe Asp Tyr
1               5                   10


<210>    28
<211>    8
<212>    PRT
<213>    Artificial Sequence

<220>
<223>    Synthetic Sequence


<400>    28
Ser Ser Asn Val Gly Gly Tyr Pro
1               5


<210>    29
<211>    3
<212>    PRT
<213>    Artificial Sequence

<220>
<223>    Synthetic Sequence


<400>    29
Thr Asp Tyr
1


<210>    30
<211>    11
<212>    PRT
<213>    Artificial Sequence

<220>
<223>    Synthetic Sequence


<400>    30
Ala Thr Trp Asp Asp Asn Leu Asn Gly Tyr Val
1               5                   10


<210>    31
<211>    360
<212>    DNA
<213>    Artificial Sequence

<220>

<223>    Synthetic Sequence

<400>    31
cagatgcagc tggtgcagtc tgggggaggc ttggtacagc ctggcaggtc cctcaaactc    60

tcctgcgcag cctctggatt ctcctttgat gattatgcca tgcactgggt ccggcaagct    120

ccagggaagg ggctggagtg ggtggccacc ataaaggacg atggaagtca gacatactat    180

gtggactctg tgaagggccg attcaccatc tccagagaca acgccaagag ctcactgttt    240

ctgcaaatga acagtctgag agccgaggac acggccgtgt attactgtac cacagaagga    300

ttaatgaatg gacttcattt tgatatgtgg ggccaaggga caatggtcac cgtctcctca    360

                                                                    360

<210>    32
<211>    120
<212>    PRT
<213>    Artificial Sequence

<220>
<223>    Synthetic Sequence

<400>    32
Gln Met Gln Leu Val Gln Ser Gly Gly Gly Leu Val Gln Pro Gly Arg
1               5                   10                  15

Ser Leu Lys Leu Ser Cys Ala Ala Ser Gly Phe Ser Phe Asp Asp Tyr
            20                  25                  30

Ala Met His Trp Val Arg Gln Ala Pro Gly Lys Gly Leu Glu Trp Val
        35                  40                  45

Ala Thr Ile Lys Asp Asp Gly Ser Gln Thr Tyr Tyr Val Asp Ser Val
    50                  55                  60

Lys Gly Arg Phe Thr Ile Ser Arg Asp Asn Ala Lys Ser Ser Leu Phe
65                  70                  75                  80

Leu Gln Met Asn Ser Leu Arg Ala Glu Asp Thr Ala Val Tyr Tyr Cys
                85                  90                  95

Thr Thr Glu Gly Leu Met Asn Gly Leu His Phe Asp Met Trp Gly Gln
            100                 105                 110

Gly Thr Met Val Thr Val Ser Ser
        115                 120

<210>    33
<211>    333
<212>    DNA
<213>    Artificial Sequence

<220>
<223>    Synthetic Sequence

<400>    33
cagctcgtgc tgactcagcc gccctcagtg tctggggccc cagggcaggg ggtcaccatc    60

tcctgcactg ggagcagctc caacatcggg gcaggttatg atgtacactg gtaccagcag    120

tttccaggaa cagcccccaa actcctcatc tctggtaaca ataatcggcc ctcagggctc    180

cctgaccgat tctctggctc caagtctggc acctcagcct ccctggccat cactggactc    240

caggctgagg atgaggctga ttattactgc cagtcctatg acagcaggct gggtgtggtc    300

ttcggcggag ggaccaagct gaccgtccta ggt                                 333


<210>    34
<211>    110
<212>    PRT
<213>    Artificial Sequence

<220>
<223>    Synthetic Sequence


<400>    34
Leu Val Leu Thr Gln Pro Pro Ser Val Ser Gly Ala Pro Gly Gln Gly
1               5                   10                  15

Val Thr Ile Ser Cys Thr Gly Ser Ser Ser Asn Ile Gly Ala Gly Tyr
            20                  25                  30

Asp Val His Trp Tyr Gln Gln Phe Pro Gly Thr Ala Pro Lys Leu Leu
        35                  40                  45

Ile Ser Gly Asn Asn Asn Arg Pro Ser Gly Leu Pro Asp Arg Phe Ser
    50                  55                  60

Gly Ser Lys Ser Gly Thr Ser Ala Ser Leu Ala Ile Thr Gly Leu Gln
65                  70                  75                  80

Ala Glu Asp Glu Ala Asp Tyr Tyr Cys Gln Ser Tyr Asp Ser Arg Leu
                85                  90                  95

Gly Val Val Phe Gly Gly Gly Thr Lys Leu Thr Val Leu Gly
            100                 105                 110


<210>    35
<211>    360
<212>    DNA
<213>    Artificial Sequence

<220>
<223>    Synthetic Sequence


<400>    35
caggtgcagc tggtggagtc tggagcagag gtgaaaagac ccggggagtc tctgaggatc    60

tcctgtaaga cttctggata cagctttacc agctactgga tccactgggt gcgccagatg    120

cccgggaaag aactggagtg gatggggagc atctatcctg ggaactctga taccagatac    180

agcccatcct tccaaggcca cgtcaccatc tcagccgaca gctccagcag caccgcctac    240

ctgcagtgga gcagcctgaa ggcctcggac accgccatgt attactgtac cacagaagga 300

ttaatgaatg gacttcattt tgatatgtgg ggccaaggga caatggtcac cgtctcctca 360

360

<210> 36
<211> 120
<212> PRT
<213> Artificial Sequence

<220>
<223> Synthetic Sequence

<400> 36
Gln Val Gln Leu Val Glu Ser Gly Ala Glu Val Lys Arg Pro Gly Glu
1               5                   10                  15

Ser Leu Arg Ile Ser Cys Lys Thr Ser Gly Tyr Ser Phe Thr Ser Tyr
            20                  25                  30

Trp Ile His Trp Val Arg Gln Met Pro Gly Lys Glu Leu Glu Trp Met
        35                  40                  45

Gly Ser Ile Tyr Pro Gly Asn Ser Asp Thr Arg Tyr Ser Pro Ser Phe
    50                  55                  60

Gln Gly His Val Thr Ile Ser Ala Asp Ser Ser Ser Ser Thr Ala Tyr
65                  70                  75                  80

Leu Gln Trp Ser Ser Leu Lys Ala Ser Asp Thr Ala Met Tyr Tyr Cys
                85                  90                  95

Thr Thr Glu Gly Leu Met Asn Gly Leu His Phe Asp Met Trp Gly Gln
            100                 105                 110

Gly Thr Met Val Thr Val Ser Ser
        115                 120

<210> 37
<211> 342
<212> DNA
<213> Artificial Sequence

<220>
<223> Synthetic Sequence

<400> 37
gatattgtga tgacccagac tccactctcc ctgcccgtca cccctggaga gccggcctcc 60

atctcctgta ggtcaagtca gagcctcctg catagtcttg gagacaatta tttggattgg 120

tatctacaga agccagggca gtctccgcaa ctcctgatct atttgggttc taagcgggcc 180

gccggggtcc ccgacaggtt cagtggcagt ggctcaggca cagactttac actcaaaatc 240

agcagagtgg aggctgagga tgttggagtt tattattgca tgcaatctct acaaactccc 300

ccgtacactt ttggccaggg gaccaagctg gagatcaaac gt 342

<210> 38
<211> 114
<212> PRT
<213> Artificial Sequence

<220>
<223> Synthetic Sequence

<400> 38
Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Leu Gly Asp Asn Tyr Leu Asp Trp Tyr Leu Gln Lys Pro Gly Gln Ser
        35                  40                  45

Pro Gln Leu Leu Ile Tyr Leu Gly Ser Lys Arg Ala Ala Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ser
                85                  90                  95

Leu Gln Thr Pro Pro Tyr Thr Phe Gly Gln Gly Thr Lys Leu Glu Ile
            100                 105                 110

Lys Arg

<210> 39
<211> 369
<212> DNA
<213> Artificial Sequence

<220>
<223> Synthetic Sequence

<400> 39
caggtgcagc tggtagagtc tgggggaggc ctggtcaagc ctggggggtc cctgagactc 60

tcctgtgcag cctctggatt caccttcagt agctatagca tgaactgggt ccgccaggct 120

ccagggaagg ggctggagtg ggtctcatcc attagtgcta gtgatggtgc cacatactac 180

gcagactccg tgaggggccg gttcaccatc tccagagaca attccaggag cacactgtat 240

ctgcaaatga acagtctgag agccgaggac acggccgtgt attactgtgc gaaaattctc 300

gcgggatata gtggcccaat gggcggaatg gacgtctggg gccaagggac cacggtcacc 360

gtctcctca 369

<210>    40
<211>    123
<212>    PRT
<213>    Artificial Sequence

<220>
<223>    Synthetic Sequence


<400>    40
Gln Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ala Ser Asp Gly Ala Thr Tyr Tyr Ala Asp Ser Val
    50                  55                  60

Arg Gly Arg Phe Thr Ile Ser Arg Asp Asn Ser Arg Ser Thr Leu Tyr
65                  70                  75                  80

Leu Gln Met Asn Ser Leu Arg Ala Glu Asp Thr Ala Val Tyr Tyr Cys
                85                  90                  95

Ala Lys Ile Leu Ala Gly Tyr Ser Gly Pro Met Gly Gly Met Asp Val
            100                 105                 110

Trp Gly Gln Gly Thr Thr Val Thr Val Ser Ser
        115                 120


<210>    41
<211>    324
<212>    PRT
<213>    Artificial Sequence

<220>
<223>    Synthetic Sequence


<400>    41
Gly Ala Cys Ala Thr Cys Cys Ala Gly Ala Thr Gly Ala Cys Cys Cys
1               5                   10                  15

Ala Gly Thr Cys Thr Cys Cys Ala Thr Cys Cys Thr Cys Ala Cys Thr
            20                  25                  30

Gly Thr Cys Thr Gly Cys Ala Thr Cys Thr Gly Thr Ala Gly Gly Ala
        35                  40                  45

Gly Ala Cys Ala Gly Ala Gly Thr Cys Ala Cys Cys Ala Thr Cys Ala
    50                  55                  60

Cys Thr Thr Gly Thr Cys Gly Gly Gly Cys Gly Ala Gly Thr Cys Gly
65                  70                  75                  80

Gly Gly Ala Cys Ala Thr Thr Ala Gly Cys Ala Ala Cys Thr Ala Thr
                85                  90                  95

Thr Thr Ala Gly Cys Cys Thr Gly Gly Thr Ala Thr Cys Ala Gly Cys
            100                 105                 110

Ala Gly Ala Ala Ala Cys Cys Ala Gly Gly Gly Ala Ala Ala Gly Cys
        115                 120                 125

Cys Cys Cys Thr Ala Ala Gly Thr Cys Cys Cys Thr Gly Ala Thr Cys
    130                 135                 140

Thr Ala Thr Gly Gly Ala Gly Cys Ala Thr Cys Cys Ala Ala Thr Thr
145                 150                 155                 160

Thr Ala Cys Ala Ala Ala Gly Thr Gly Gly Gly Gly Thr Cys Thr Cys
                165                 170                 175

Ala Thr Cys Ala Cys Ala Gly Thr Thr Cys Ala Gly Cys Gly Gly Cys
            180                 185                 190

Ala Gly Thr Gly Gly Ala Thr Cys Cys Gly Gly Gly Ala Cys Ala Gly
        195                 200                 205

Ala Thr Thr Thr Cys Ala Cys Cys Cys Thr Cys Ala Cys Cys Ala Thr
    210                 215                 220

Cys Ala Ala Cys Ala Gly Cys Cys Thr Gly Cys Ala Gly Cys Cys Thr
225                 230                 235                 240

Gly Ala Ala Gly Ala Thr Thr Cys Thr Gly Cys Ala Ala Cys Thr Thr
                245                 250                 255

Ala Thr Thr Ala Cys Thr Gly Thr Cys Ala Ala Cys Ala Gly Thr Ala
            260                 265                 270

Cys Thr Ala Thr Ala Gly Thr Thr Ala Cys Cys Cys Gly Cys Thr Cys
        275                 280                 285

Ala Cys Thr Thr Thr Thr Gly Gly Cys Gly Gly Ala Gly Gly Gly Ala
    290                 295                 300

Cys Cys Ala Ala Gly Gly Thr Gly Gly Ala Thr Ala Thr Cys Ala Ala
305                 310                 315                 320

Ala Cys Gly Thr

<210>  42
<211>  108
<212>  PRT
<213>  Artificial Sequence

<220>
<223>  Synthetic Sequence

<400>  42
Asp Ile Gln Met Thr Gln Ser Pro Ser Ser Leu Ser Ala Ser Val Gly
1               5                   10                  15

Asp Arg Val Thr Ile Thr Cys Arg Ala Ser Arg Asp Ile Ser Asn Tyr
            20                  25                  30

Leu Ala Trp Tyr Gln Gln Lys Pro Gly Lys Ala Pro Lys Ser Leu Ile

        35                  40                  45

Tyr Gly Ala Ser Asn Leu Gln Ser Gly Val Ser Ser Gln Phe Ser Gly
    50                  55                  60

Ser Gly Ser Gly Thr Asp Phe Thr Leu Thr Ile Asn Ser Leu Gln Pro
65                  70                  75                  80

Glu Asp Ser Ala Thr Tyr Tyr Cys Gln Gln Tyr Tyr Ser Tyr Pro Leu
                85                  90                  95

Thr Phe Gly Gly Gly Thr Lys Val Asp Ile Lys Arg
            100                 105

<210> 43
<211> 360
<212> DNA
<213> Artificial Sequence

<220>
<223> Synthetic Sequence

<400> 43
caggtgcagc tggtggagtc tgggggaggc ttggtacagc ctggagggtc cctgagactc 60

tcctgtgaag cctctggatt cgccttcggt cgttatgaga tgaattgggt ccgccaggct 120

ccagggaagg ggctggagtg gattgcatat attgatactg gtggtggtgc caaagtctat 180

gcagactctg tgaagggccg attcaccatc tccagagacg acagcaaaaa ctccctgtat 240

ctgcaaatga acagtctgag agccgaggac acggccgtgt attactgtac cacagaagga 300

ttaatgaatg gacttcattt tgatatgtgg ggccaaggga caatgatcac cgtctcctca 360

360

<210> 44
<211> 120
<212> PRT
<213> Artificial Sequence

<220>
<223> Synthetic Sequence

<400> 44
Gln Val Gln Leu Val Glu Ser Gly Gly Gly Leu Val Gln Pro Gly Gly
1               5                   10                  15

Ser Leu Arg Leu Ser Cys Glu Ala Ser Gly Phe Ala Phe Gly Arg Tyr
            20                  25                  30

Glu Met Asn Trp Val Arg Gln Ala Pro Gly Lys Gly Leu Glu Trp Ile
        35                  40                  45

Ala Tyr Ile Asp Thr Gly Gly Gly Ala Lys Val Tyr Ala Asp Ser Val
    50                  55                  60

Lys Gly Arg Phe Thr Ile Ser Arg Asp Asp Ser Lys Asn Ser Leu Tyr

65                  70                  75                  80

Leu Gln Met Asn Ser Leu Arg Ala Glu Asp Thr Ala Val Tyr Tyr Cys
                85                  90                  95

Thr Thr Glu Gly Leu Met Asn Gly Leu His Phe Asp Met Trp Gly Gln
            100                 105                 110

Gly Thr Met Ile Thr Val Ser Ser
        115                 120


<210>    45
<211>    324
<212>    DNA
<213>    Artificial Sequence

<220>
<223>    Synthetic Sequence


<400>    45
gacatccaga tgacccagtc tccttccacc ctgtctgcat ctgtaggaga cagagtcgcc      60

atcacttgcc gggccagtca ggctattagt acctggttgg cctggtatca gcagaaacct     120

ggtaaagccc ctaaactcct gatctatacg gcgtctactt tagaaagtgg ggtcccatca     180

aggttcagcg gcagtggatc tgggacagat ttcactctca ccatcaacag cctgcagcct     240

gatgattttg caacttatta ctgtcaacag cttaatagtt acccttacac tttcggcgga     300

gggaccaagg tggagatcaa acgt                                            324


<210>    46
<211>    108
<212>    PRT
<213>    Artificial Sequence

<220>
<223>    Synthetic Sequence


<400>    46
Asp Ile Gln Met Thr Gln Ser Pro Ser Thr Leu Ser Ala Ser Val Gly
1               5                   10                  15

Asp Arg Val Ala Ile Thr Cys Arg Ala Ser Gln Ala Ile Ser Thr Trp
            20                  25                  30

Leu Ala Trp Tyr Gln Gln Lys Pro Gly Lys Ala Pro Lys Leu Leu Ile
        35                  40                  45

Tyr Thr Ala Ser Thr Leu Glu Ser Gly Val Pro Ser Arg Phe Ser Gly
    50                  55                  60

Ser Gly Ser Gly Thr Asp Phe Thr Leu Thr Ile Asn Ser Leu Gln Pro
65                  70                  75                  80

Asp Asp Phe Ala Thr Tyr Tyr Cys Gln Gln Leu Asn Ser Tyr Pro Tyr
                85                  90                  95

Thr Phe Gly Gly Gly Thr Lys Val Glu Ile Lys Arg
            100                 105

<210>    47
<211>    354
<212>    DNA
<213>    Artificial Sequence

<220>
<223>    Synthetic Sequence

<400>    47
caggtgcagc tggtggagtc tgggggaggc ttggtacagc ctggcaggtc cctgagactc        60

tcctgtgcag cctctggatt cacctttgat gattgtgcca tgcactgggt ccgacaagct       120

ccagggaagg gcctggagtg ggtctcaggt attagtggga atagtaaaaa cgtagcctat       180

gcggactctg tgaagggccg attcagcatc tccagagacg acgccaagaa ctccctgtat       240

ctgcaaatga acagtctgag agccgaggac acggccgtgt attactgtgc gagagatccg       300

gcatacagcc agtttgacta ctggggccag ggaaccctga tcaccgtctc ctca             354

<210>    48
<211>    118
<212>    PRT
<213>    Artificial Sequence

<220>
<223>    Synthetic Sequence

<400>    48
Gln Val Gln Leu Val Glu Ser Gly Gly Gly Leu Val Gln Pro Gly Arg
 1               5                  10                  15

Ser Leu Arg Leu Ser Cys Ala Ala Ser Gly Phe Thr Phe Asp Asp Cys
            20                  25                  30

Ala Met His Trp Val Arg Gln Ala Pro Gly Lys Gly Leu Glu Trp Val
        35                  40                  45

Ser Gly Ile Ser Gly Asn Ser Lys Asn Val Ala Tyr Ala Asp Ser Val
    50                  55                  60

Lys Gly Arg Phe Ser Ile Ser Arg Asp Asp Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Pro Ala Tyr Ser Gln Phe Asp Tyr Trp Gly Gln Gly Thr
            100                 105                 110

Leu Ile Thr Val Ser Ser
        115

<210>    49

<211>    333
<212>    DNA
<213>    Artificial Sequence

<220>
<223>    Synthetic Sequence


<400>    49
cagtctgccc tgactcagcc tccctcactg tctgcgaccc ccgggcagag ggtcaccatc        60

tcttgctctg gaagcagctc caacgtcgga ggatatcctg tcaactggta ccagcaggtc       120

ccaggagcgg cccccaaact cctcatgtat actgattata agcggccctc aggtgtccct       180

gaccgattct ttggctccaa gtctggcact tcagcctccc tggccatcag tggcctccag       240

tctgaagatg aggctgatta ttactgtgct acatgggacg acaacctgaa tggctatgtc       300

ttcggaactg ggaccaaggt caccgtccta ggt                                    333


<210>    50
<211>    111
<212>    PRT
<213>    Artificial Sequence

<220>
<223>    Synthetic Sequence


<400>    50
Gln Ser Ala Leu Thr Gln Pro Pro Ser Leu Ser Ala Thr Pro Gly Gln
1               5                   10                  15

Arg Val Thr Ile Ser Cys Ser Gly Ser Ser Ser Asn Val Gly Gly Tyr
            20                  25                  30

Pro Val Asn Trp Tyr Gln Gln Val Pro Gly Ala Ala Pro Lys Leu Leu
        35                  40                  45

Met Tyr Thr Asp Tyr Lys Arg Pro Ser Gly Val Pro Asp Arg Phe Phe
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Thr Trp Asp Asp Asn Leu
                85                  90                  95

Asn Gly Tyr Val Phe Gly Thr Gly Thr Lys Val Thr Val Leu Gly
            100                 105                 110




<210>    51
<211>    16
<212>    PRT
<213>    Artificial Sequence

<220>
<223>    Synthetic Sequence

<400>    51
Ala Lys Thr Leu Ala Gly Tyr Ser Gly Pro Met Gly Gly Met Asp Val
1               5                   10                  15

<210>    52
<211>    16
<212>    PRT
<213>    Artificial Sequence

<220>
<223>    Synthetic Sequence

<400>    52
Ala Lys Ile Leu Val Gly Tyr Ser Gly Pro Met Gly Gly Met Asp Val
1               5                   10                  15

<210>    53
<211>    16
<212>    PRT
<213>    Artificial Sequence

<220>
<223>    Synthetic Sequence

<400>    53
Ala Lys Ser Leu Ala Ser Tyr Ser Gly Pro Met Gly Gly Met Asp Val
1               5                   10                  15

<210>    54
<211>    369
<212>    DNA
<213>    Artificial Sequence

<220>
<223>    Synthetic Sequence

<400>    54
caggtgcagc tggtagagtc tgggggaggc ctggtcaagc ctggggggtc cctgagactc      60

tcctgtgcag cctctggatt caccttcagt agctatagca tgaactgggt ccgccaggct      120

ccagggaagg ggctggagtg ggtctcatcc attagtgcta gtgatggtgc cacatactac      180

gcagactccg tgaggggccg gttcaccatc tccagagaca attccaggag cacactgtat      240

ctgcaaatga acagtctgag agccgaggac acggccgtgt attactgtgc gaaaactctc      300

gcgggatata gtggcccaat gggtggcatg gacgtctggg gccaagggac cacggtcacc      360

gtctcctca                                                          369

<210>    55
<211>    123
<212>    PRT
<213>    Artificial Sequence

<220>
<223>    Synthetic Sequence

<400>    55
Gln Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ala Ser Asp Gly Ala Thr Tyr Tyr Ala Asp Ser Val
    50                  55                  60

Arg Gly Arg Phe Thr Ile Ser Arg Asp Asn Ser Arg Ser Thr Leu Tyr
65                  70                  75                  80

Leu Gln Met Asn Ser Leu Arg Ala Glu Asp Thr Ala Val Tyr Tyr Cys
                85                  90                  95

Ala Lys Thr Leu Ala Gly Tyr Ser Gly Pro Met Gly Gly Met Asp Val
            100                 105                 110

Trp Gly Gln Gly Thr Thr Val Thr Val Ser Ser
        115                 120

<210>    56
<211>    369
<212>    DNA
<213>    Artificial Sequence

<220>
<223>    Synthetic Sequence

<400>    56
caggtgcagc tggtagagtc tgggggaggc ctggtcaagc ctggggggtc cctgagactc      60

tcctgtgcag cctctggatt caccttcagt agctatagca tgaactgggt ccgccaggct     120

ccagggaagg ggctggagtg ggtctcatcc attagtgcta gtgatggtgc cacatactac     180

gcagactccg tgaggggccg gttcaccatc tccagagaca attccaggag cacactgtat     240

ctgcaaatga acagtctgag agccgaggac acggccgtgt attactgtgc gaaaatcctc     300

gtaggataca gtggcccaat gggcggaatg gacgtctggg gccaagggac cacggtcacc     360

gtctcctca                                                          369

<210>    57
<211>    123
<212>    PRT
<213>    Artificial Sequence

<220>
<223>    Synthetic Sequence


<400>    57
Gln Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ala Ser Asp Gly Ala Thr Tyr Tyr Ala Asp Ser Val
    50                  55                  60

Arg Gly Arg Phe Thr Ile Ser Arg Asp Asn Ser Arg Ser Thr Leu Tyr
65                  70                  75                  80

Leu Gln Met Asn Ser Leu Arg Ala Glu Asp Thr Ala Val Tyr Tyr Cys
                85                  90                  95

Ala Lys Ile Leu Val Gly Tyr Ser Gly Pro Met Gly Gly Met Asp Val
            100                 105                 110

Trp Gly Gln Gly Thr Thr Val Thr Val Ser Ser
        115                 120


<210>    58
<211>    369
<212>    DNA
<213>    Artificial Sequence

<220>
<223>    Synthetic Sequence


<400>    58
caggtgcagc tggtagagtc tgggggaggc ctggtcaagc ctggggggtc cctgagactc      60

tcctgtgcag cctctggatt caccttcagt agctatagca tgaactgggt ccgccaggct     120

ccagggaagg ggctggagtg ggtctcatcc attagtgcta gtgatggtgc cacatactac     180

gcagactccg tgaggggccg gttcaccatc tccagagaca attccaggag cacactgtat     240

ctgcaaatga acagtctgag agccgaggac acggccgtgt attactgtgc gaaaagcctt     300

gccagctata gtggcccaat gggcggcatg gacgtctggg gccaagggac cacggtcacc     360

gtctcctca                                                             369


<210>    59
<211>    123
<212>    PRT

<213>    Artificial Sequence

<220>
<223>    Synthetic Sequence


<400>    59
Gln Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ala Ser Asp Gly Ala Thr Tyr Tyr Ala Asp Ser Val
    50                  55                  60

Arg Gly Arg Phe Thr Ile Ser Arg Asp Asn Ser Arg Ser Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Leu Ala Ser Tyr Ser Gly Pro Met Gly Gly Met Asp Val
            100                 105                 110

Trp Gly Gln Gly Thr Thr Val Thr Val Ser Ser
        115                 120


<210>    60
<211>    369
<212>    DNA
<213>    Artificial Sequence

<220>
<223>    Synthetic Sequence


<400>    60
caggtgcagc tggtagagtc tgggggaggc ctgttcaagc ctggggggtc cctgagactc      60

tcctgtgcag cctctggatt caccttcagt agctatagca tgaactgggt ccgccaggct      120

ccagggaagg ggctggagtg ggtctcatcc attagtgcta gtgatggtgc cacatactac      180

gcagactccg tgaggggccg gttcaccatc tccagagaca attccaggag cacactgtat      240

ctgcaaatga acagtctgag agccgaggac acggccgtgt attactgtgc gaaaactctc      300

gcgggatata gtggcccaat gggtggcatg gacgtctggg gccaagggac cacggtcacc      360

gtctcctca                                                              369


<210>    61
<211>    123
<212>    PRT
<213>    Artificial Sequence

<220>

<223>    Synthetic Sequence

<400>    61
Gln Val Gln Leu Val Glu Ser Gly Gly Gly Leu Ser Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ala Ser Asp Gly Ala Thr Tyr Tyr Ala Asp Ser Val
    50                  55                  60

Arg Gly Arg Phe Thr Ile Ser Arg Asp Asn Ser Arg Ser Thr Leu Tyr
65                  70                  75                  80

Leu Gln Met Asn Ser Leu Arg Ala Glu Asp Thr Ala Val Tyr Tyr Cys
                85                  90                  95

Ala Lys Thr Leu Ala Gly Tyr Ser Gly Pro Met Gly Gly Met Asp Val
            100                 105                 110

Trp Gly Gln Gly Thr Thr Val Thr Val Ser Ser
        115                 120